# EUROPEAN PATENT APPLICATION

(11) **EP 4 692 804 A1**
(43) Date of publication of application: **11.02.2026**
(21) Application number: 24784638.9
(22) Date of filing: 27.02.2024
(51) Int. Cl.: G01N 35/00, C12M 1/34, G01N 33/537

(54) **SAMPLE ANALYZER**

(30) Priority: 05.04.2023 JP 2023061090
(71) Applicant: HITACHI HIGH-TECH CORPORATION, Tokyo 105-6409 (JP)
(72) Inventor: MATSUZAWA, Mitsuhiro, Tokyo 100-8280 (JP); NODA, Kazuhiro, Tokyo 100-8280 (JP); SUGIMURA, Yoshiaki, Tokyo 105-6409 (JP)
(74) Representative: MERH-IP Matias Erny Reichl Hoffmann Patentanwälte PartG mbB
(86) International application number: PCT/JP2024/007175
(87) International publication number: WO 2024/209842

(57) **Abstract**

To increase capture efficiency of magnetic particles at a predetermined position and to perform detection with high sensitivity, the disclosure proposes a sample analyzer including: a flow channel including a capture region defined therein and configured to introduce, into the capture region, a sample liquid containing magnetic particles bound to a specific substance; a supply unit configured to supply the sample liquid to the flow channel; a capture unit configured to generate a magnetic field and cause the magnetic particles to be adsorbed to the capture region by the magnetic field; a magnetic particle distribution adjustment unit configured to make, for a distribution of the magnetic particles in a cross section perpendicular to a flow direction of the sample liquid in the capture region of the flow channel, a distribution in a proximal region closer to the capture region than a center axis of the flow channel denser than a distribution in a distal region farther from the central axis as viewed from the capture region; a measurement unit configured to measure the specific substance adsorbed to the capture region; and a discharge unit configured to discharge the magnetic particles from the flow channel after measurement by the measurement unit (see FIG. 9).

## Description

### Technical Field

The present disclosure relates to a sample analyzer.

### Background Art

First, immunoanalysis is described as an example of sample analysis. The immunoanalysis refers to detection or measurement of an antibody or an antigen in a body fluid (such as plasma, serum, or urine) using a specific reaction between the antigen and the antibody for a purpose of diagnosing a disease or diagnosing a pathological condition. As a typical method, there is an enzyme-linked immunosorbent assay (ELISA) method.

As a typical implementation process of the ELISA method, an antibody (first antibody) against an antigen to be measured is immobilized in a container, a sample such as plasma, serum, or urine is placed therein, and an antigen in the sample is allowed to bind to the first antibody. An antibody (second antibody) bound to a label is further allowed to bind to the antigen bound to the first antibody. By collecting a complex of the first antibody, the antigen, the second antibody, and the label, and detecting a signal emitted from the label, presence or absence and an amount of the antigen in the sample are measured.

For example, a fluorescent substance is used as the label. In this case, light emission is stronger in proportion to the number of second antibodies bound to the label, that is, the amount of antigen in the complex. By detecting the light emission of the fluorescent substance with a photomultiplier or the like, the antigen in the sample can be quantified.

A specific example of an immunoassay device using the ELISA method will be described. In this example, magnetic particles are used as a solid phase, and the first antibody is immobilized on surfaces of the magnetic particles. A fluorochrome-labeled substance containing a fluorescent dye as a label is bound to the second antibody. By mixing a biologically derived detection substance (antigen) and the magnetic particles on which the first antibody is immobilized to cause an antigen-antibody reaction, a specific antigen contained in the sample binds to the magnetic particles via the first antibody. When the second antibody is further made to react, the fluorochrome-labeled substance binds to the magnetic particles via the second antibody, the antigen, and the first antibody. An amount of the fluorochrome-labeled substance increases or decreases depending on an amount of a detection substance contained in the sample, that is, an amount of the antigen.

The magnetic particles are captured (adsorbed) at a predetermined position in a flow channel while a liquid sample containing the magnetic particles to which the detection substance is bound is caused to flow through any flow channel. By applying an external electric field or the like to the captured magnetic particles, the fluorochrome-labeled substance bound to the magnetic particles emits light. By detecting an intensity of the emitted light at this time, the amount of the detection substance in the sample, that is, the amount of the antigen can be measured, and quantitative measurement is performed.

To perform highly sensitive immunoanalysis, the magnetic particles to which the detection substance (antigen) is bound is captured at a specific location using a magnet or the like, and so-called bound/free separation (B/F separation, separation of an antigen-antibody complex and unbound components) is performed to replace a solution containing an antibody that is not bound to the antigen.

For example, PTL 1 discloses a method of capturing magnetic particles at a predetermined position using a magnet or the like in an analyzer. Specifically, PTL 1 describes a sample analyzer that includes at least one of a configuration in which a flow channel cross-sectional area at a downstream end of a magnetic particle capture region is greater than a flow channel cross-sectional area at an upstream end of the magnetic particle capture region, and a configuration in which a magnitude of a magnetic field generated by a magnetic field generation unit is greater on a downstream side than on an upstream side of the magnetic particle capture region, and this configuration can prevent non-uniformity of adsorption.

### Citation List

### Patent Literature

PTL 1: WO2011/155489A

### Summary of Invention

### Technical Problem

However, in the sample analyzer disclosed in PTL 1, some of the introduced magnetic particles may flow out without being captured, and sensitivity may decrease.

In view of such circumstances, the disclosure provides a sample analysis technique that increases capture efficiency of magnetic particles at a predetermined position and performs detection with high sensitivity.

### Solution to Problem

To solve the above problems, the disclosure proposes, as a representative sample analyzer, a sample analyzer including: a flow channel including a capture region defined therein and configured to introduce, into the capture region, a sample liquid containing magnetic particles bound to a specific substance; a supply unit configured to supply the sample liquid to the flow channel; a capture unit configured to generate a magnetic field and cause the magnetic particles to be adsorbed to the capture region by the magnetic field; a magnetic particle distribution adjustment unit configured to make, for a distribution of the magnetic particles in a cross section perpendicular to a flow direction of the sample liquid in the capture region of the flow channel, a distribution in a proximal region closer to the capture region than a center axis of the flow channel denser than a distribution in a distal region farther from the central axis as viewed from the capture region; a measurement unit configured to measure the specific substance adsorbed to the capture region; and a discharge unit configured to discharge the magnetic particles from the flow channel after measurement by the measurement unit.

Additional features related to the disclosure will be clarified from the description of the present description and the accompanying drawings. Aspects of the disclosure may be achieved and implemented using elements, combinations of various elements, the following detailed description, and accompanying claims.

The description of the present description is merely a typical illustration and does not limit the scope of the claims or application examples of the disclosure in any sense.

### Advantageous Effects of Invention

According to the technique of the disclosure, in the sample analyzer, capture efficiency of magnetic particles at a predetermined position can be increased, and detection can be performed with high sensitivity.

### Brief Description of Drawings

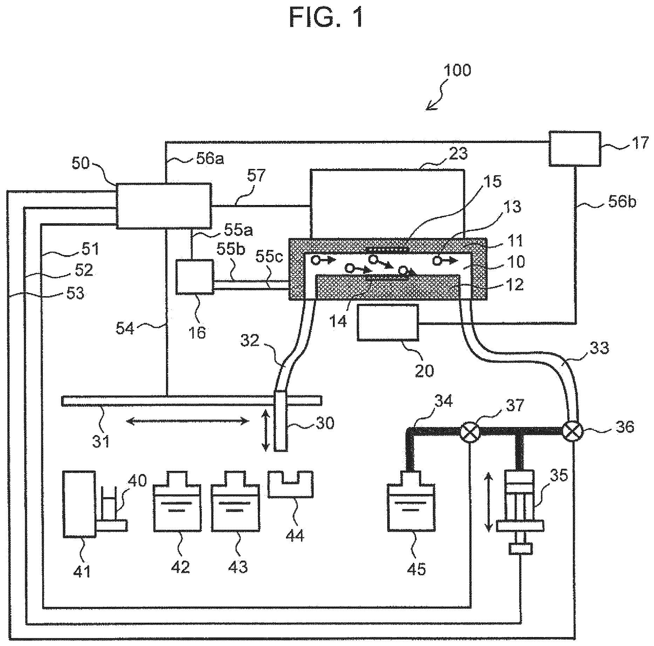

[FIG. 1] FIG. 1 is a diagram illustrating a schematic configuration example of an immunoanalyzer 100 according to the present embodiment.

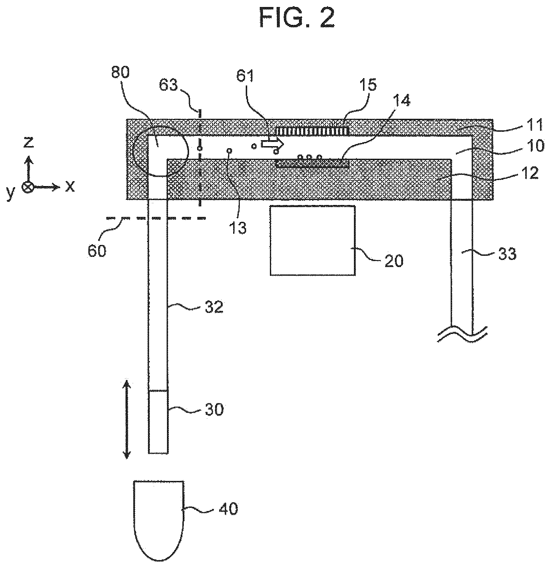

[FIG. 2] FIG. 2 is a diagram illustrating a flow cell and a magnetic field structure according to an existing configuration example using a permanent magnet.

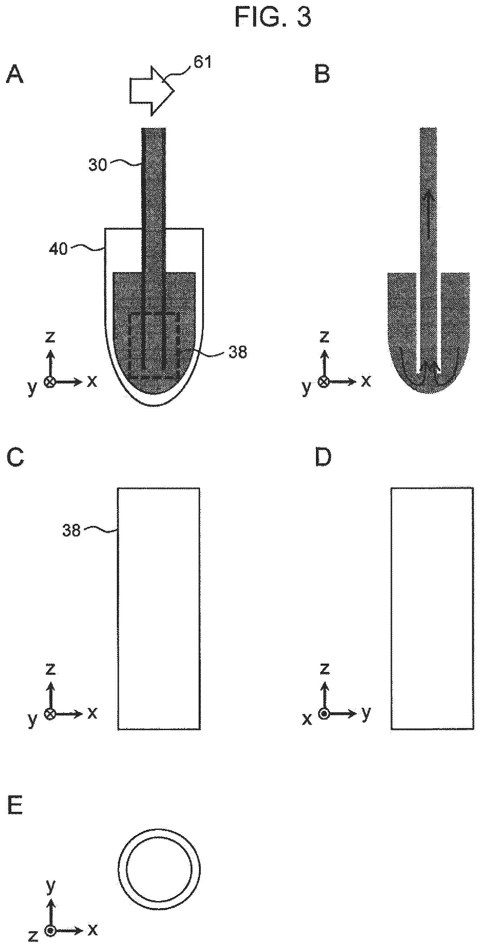

[FIG. 3] A of FIG. 3 is a diagram illustrating a position relation between a sipper nozzle 30 and a suspension container 40. B of FIG. 3 is a diagram illustrating a fluid region for which a calculation is performed in fluid analysis. C of FIG. 3 is a diagram illustrating a cross section taken along a plane that passes through a center axis of the sipper nozzle 30 and is parallel to a zx plane in a sipper nozzle distal end portion 38. D of FIG. 3 is a diagram illustrating a cross section taken along a plane that passes through the center axis of the sipper nozzle 30 and is parallel to a yz plane. E of FIG. 3 is a diagram illustrating a cross section taken along a plane parallel to an xy plane of the sipper nozzle 30.

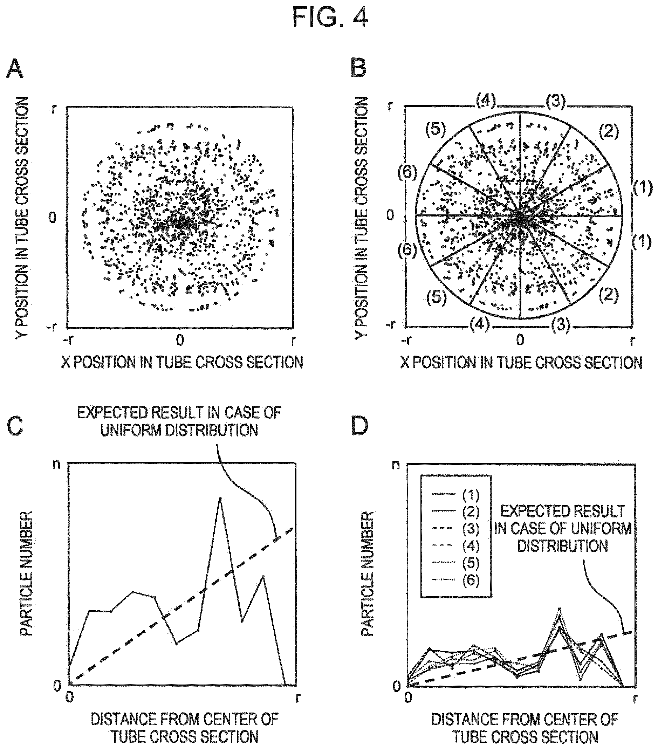

[FIG. 4] A of FIG. 4 is a diagram illustrating a magnetic particle distribution in a cross section 60 in FIG. 2 in an existing immunoanalyzer. B of FIG. 4 is a diagram illustrating a state in which a region in A of FIG. 4 is divided into 12 in a circumferential direction. C of FIG. 4 is a graph illustrating a relation between a distance from a center and a particle number in the cross section 60 when the axis of the sipper nozzle 30 is the center. D of FIG. 4 is a graph illustrating a relation between a distance from a center of the sipper nozzle and a particle number in each region divided in B of FIG. 4.

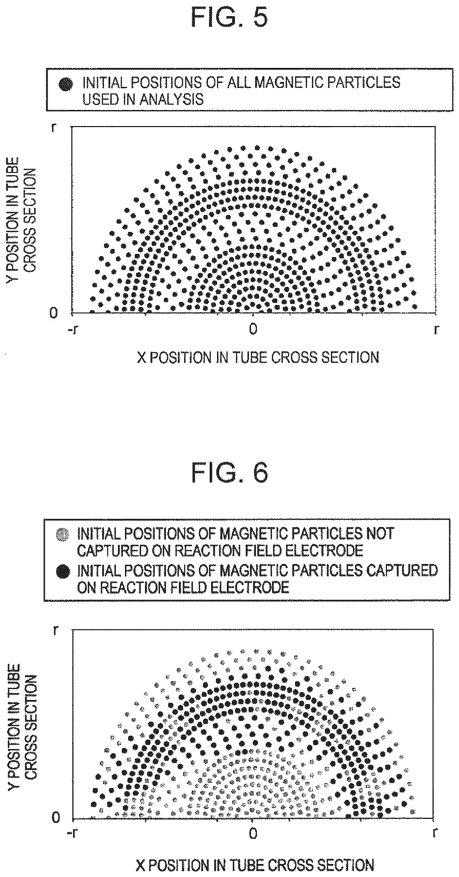

[FIG. 5] FIG. 5 is a diagram illustrating a distribution of magnetic particles 13 in the cross section 60 of the sipper nozzle 30 based on results in A and C of FIG. 4.
[FIG. 6] FIG. 6 is a diagram illustrating that when a fluid-magnetic field analysis in which the magnetic particles 13 are captured is performed by an immunoanalyzer in an existing example, only magnetic particles in a region illustrated in black are captured on a reaction field electrode 14, and magnetic particles in a region illustrated in gray pass through a flow channel 10 and flow out to a tube 33 without being captured on the reaction field electrode 14.

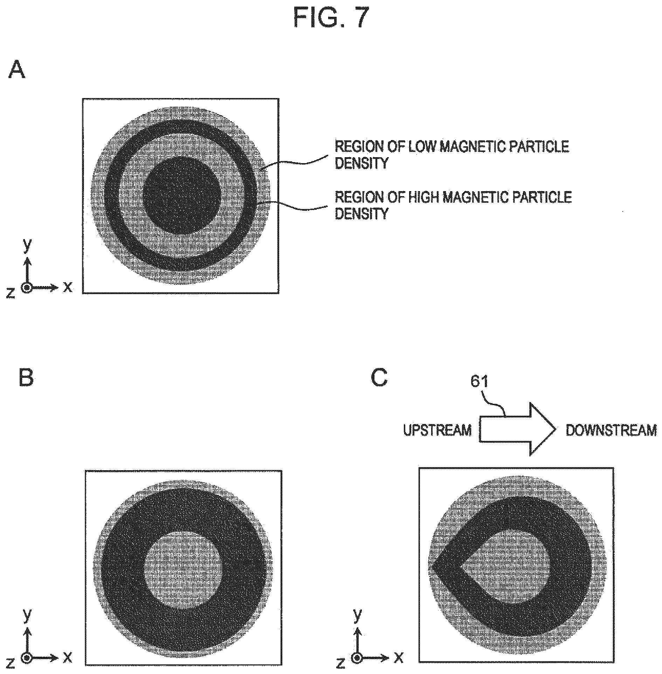

[FIG. 7] A of FIG. 7 is a diagram illustrating a schematic diagram of the magnetic particle distribution in the cross section 60 in the existing configuration example illustrated in FIGS. 2 to 6. B of FIG. 7 is a schematic diagram of magnetic particle distribution Example 1 in the cross section 60 achieved in the embodiment of the disclosure. C of FIG. 7 is a schematic diagram of magnetic particle distribution Example 2 in the cross section 60 achieved in the embodiment of the disclosure.

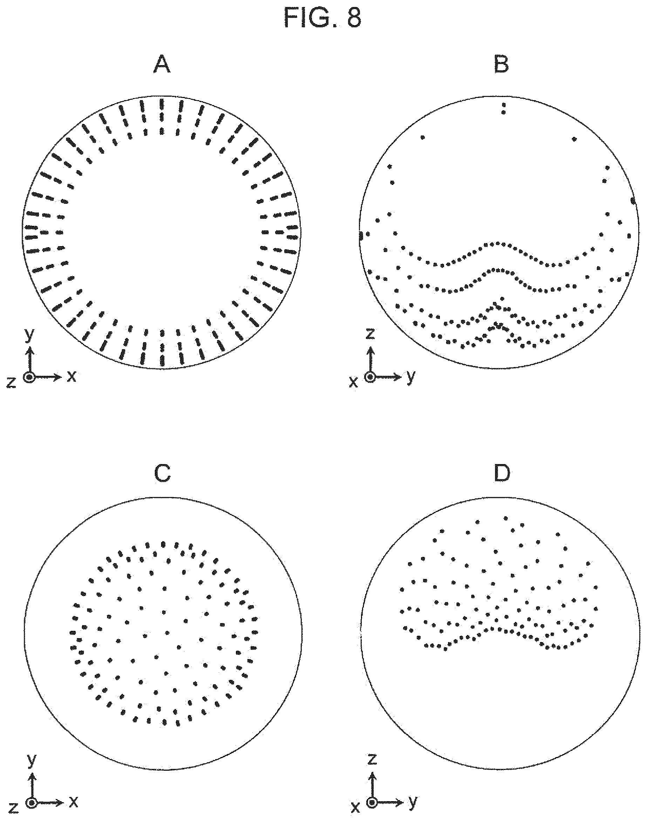

[FIG. 8] A of FIG. 8 is a diagram illustrating an initial state of an analysis in which magnetic particles are distributed outside a flow channel in the cross section 60. B of FIG. 8 is a diagram illustrating a magnetic particle distribution in the cross section 63 after passing through a bent portion 80 from the initial state in A of FIG. 8. C of FIG. 8 is a diagram illustrating a state in which magnetic particles are distributed on an inner side of the flow channel in the cross section 60. D of FIG. 8 is a diagram illustrating a magnetic particle distribution in the cross section 63 after passing through the bent portion 80 from an initial state in C of FIG. 8.

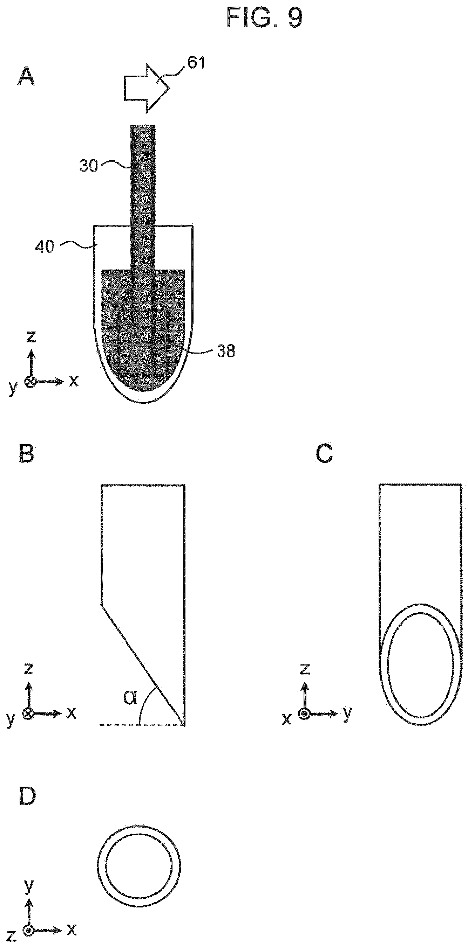

[FIG. 9] A of FIG. 9 illustrates a position relation between the sipper nozzle 30 and the suspension container 40. B of FIG. 9 is a diagram illustrating a cross-sectional configuration taken along a plane that passes through a center axis of the sipper nozzle 30 and is parallel to a zx plane in a distal end portion 38 of the sipper nozzle 30. C of FIG. 9 is a diagram illustrating a cross-sectional configuration taken along a plane that passes through the center axis of the sipper nozzle 30 and is parallel to a yz plane. D of FIG. 9 is a diagram illustrating a cross-sectional configuration taken along a plane parallel to an xy plane of the sipper nozzle 30.

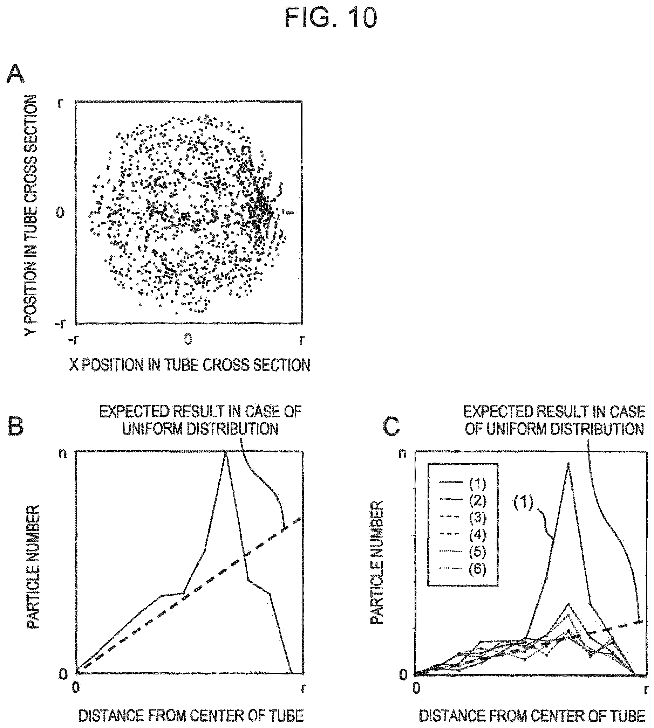

[FIG. 10] A of FIG. 10 is a diagram illustrating a magnetic particle distribution in the cross section 60 when the injection needle-shaped sipper nozzle 30 according to Example 1 is used. B of FIG. 10 is a graph illustrating a relation between a distance from a center and a particle number in the cross section 60 when an axis of the sipper nozzle 30 is the center. C of FIG. 10 is a graph illustrating a relation between a distance from the center and a particle number in each region divided in a circumferential direction as in D of FIG. 4.

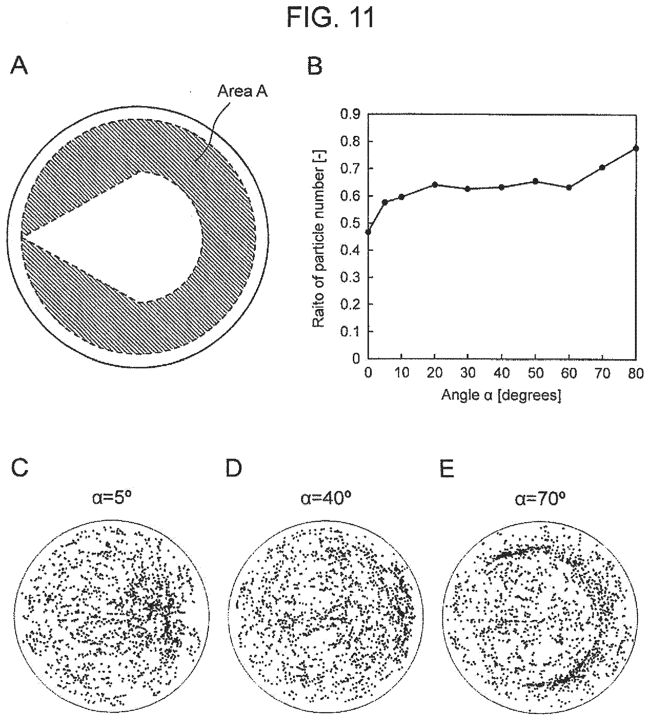

[FIG. 11] A of FIG. 11 is a diagram illustrating Area A (hatched region) in the magnetic particle distribution in the cross section 60 of the sipper nozzle 30. B of FIG. 11 is a diagram illustrating a ratio of particles distributed in Area A when an angle α of the sipper nozzle 30 (see B of FIG. 9) is changed. C of FIG. 11 is a diagram illustrating a particle distribution in the cross section 60 when the angle α = 5°. D of FIG. 11 is a diagram illustrating a particle distribution in the cross section 60 when the angle α = 40°. E of FIG. 11 is a diagram illustrating a particle distribution in the cross section 60 when the angle α = 70°.

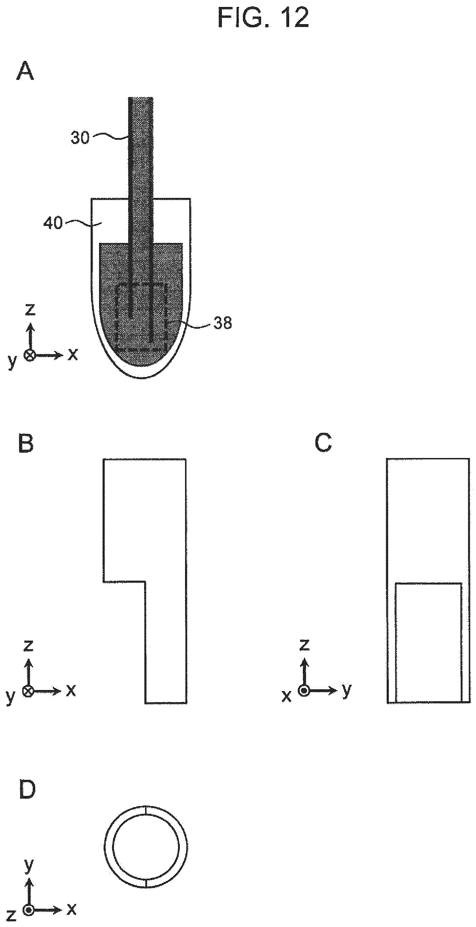

[FIG. 12] A of FIG. 12 is a diagram illustrating a position relation between the sipper nozzle 30 according to Modification 1 of Example 1 and the suspension container 40. B of FIG. 12 is a diagram illustrating a cross section taken along a plane that passes through a center axis of the sipper nozzle 30 according to Modification 1 and is parallel to a zx plane in the cross section 60. C of FIG. 12 is a diagram illustrating a cross section taken along a plane that passes through the center axis of the sipper nozzle 30 according to Modification 1 and is parallel to a yz plane. D of FIG. 12 is a diagram illustrating a cross section taken along a plane parallel to an xy plane of the sipper nozzle 30 according to Modification 1.

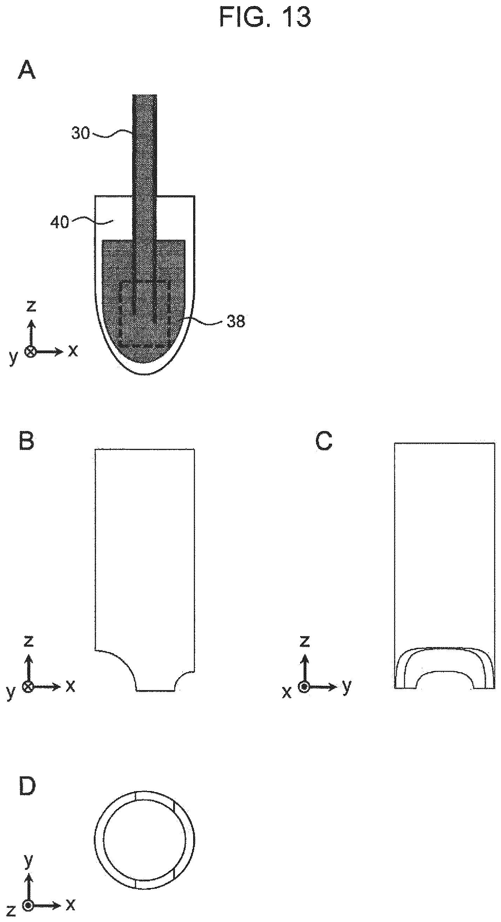

[FIG. 13] A of FIG. 13 is a diagram illustrating a position relation between the sipper nozzle 30 according to Modification 2 of Example 1 and the suspension container 40. B of FIG. 13 is a diagram illustrating a cross section taken along a plane that passes through a center axis of the sipper nozzle 30 according to Modification 2 and is parallel to a zx plane in the cross section 60. C of FIG. 13 is a diagram illustrating a cross section taken along a plane that passes through the center axis of the sipper nozzle 30 according to Modification 2 and is parallel to a yz plane. D of FIG. 13 is a diagram illustrating a cross section taken along a plane parallel to an xy plane of the sipper nozzle 30 according to Modification 2.

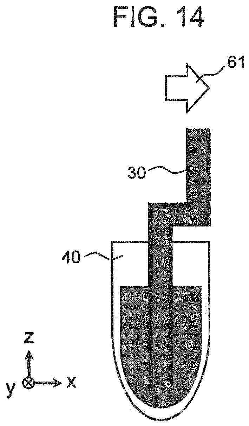

[FIG. 14] FIG. 14 illustrates a position relation between the sipper nozzle 30 according to Example 2 and the suspension container 40.

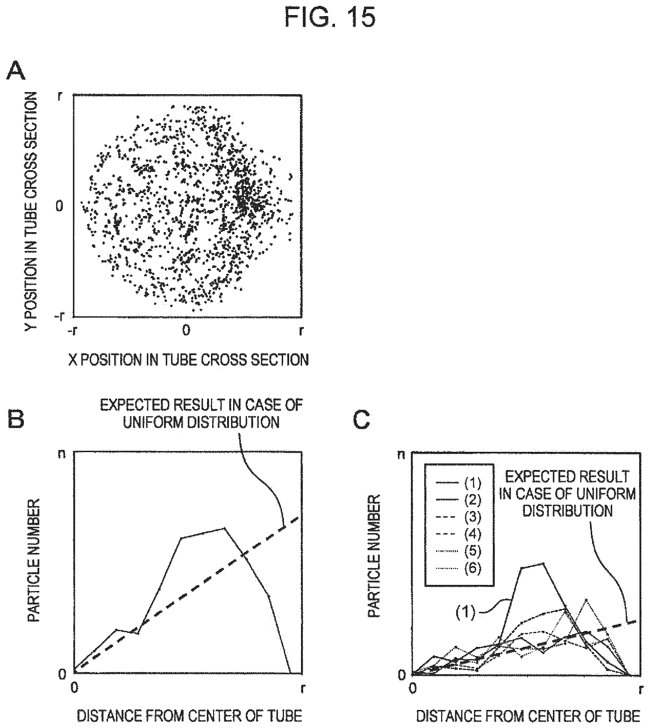

[FIG. 15] A of FIG. 15 is a diagram illustrating a magnetic particle distribution in the cross section 60 in Example 2. B of FIG. 15 is a graph illustrating a relation between a distance from a center and a particle number in the cross section 60 when an axis of the sipper nozzle 30 is the center in Example 2. C of FIG. 15 is a graph illustrating a relation between a distance from the center of the sipper nozzle 30 and a particle number in each divided region in Example 2, similarly to D of FIG. 4.

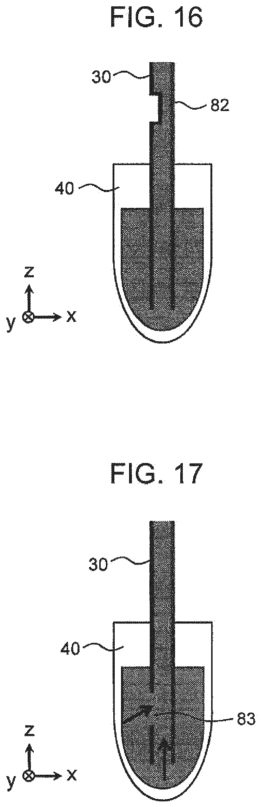

[FIG. 16] FIG. 16 illustrates a configuration example of the sipper nozzle 30 according to Example 3, in which a flow contraction portion 82 is provided midway along the flow channel such that the flow channel narrows in an x direction.
[FIG. 17] FIG. 17 is a diagram illustrating a configuration example of the sipper nozzle 30 according to Example 3, in which a hole 83 is formed on a side surface midway along the flow channel in the x direction.

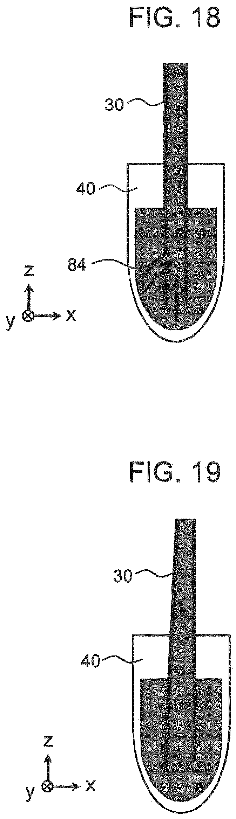

[FIG. 18] FIG. 18 is a diagram illustrating a configuration example of the sipper nozzle 30 according to Example 3, in which a branch flow channel 84 is connected to the side surface midway along the flow channel in the x direction.
[FIG. 19] FIG. 19 is a diagram illustrating a configuration example of the sipper nozzle 30 according to Example 3, in the sipper nozzle 30, a cross-sectional area in a plane parallel to an xy plane decreases from upstream to downstream, and a flow channel center in each cross section is formed to move in a +x direction from upstream to downstream.

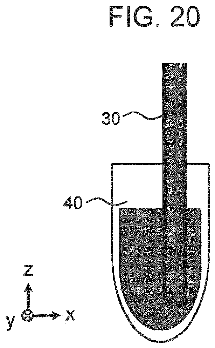

[FIG. 20] FIG. 20 is a diagram illustrating Example 4, in which the center axis of the sipper nozzle 30 is disposed offset from a center axis of the suspension container 40 in an x axis direction.

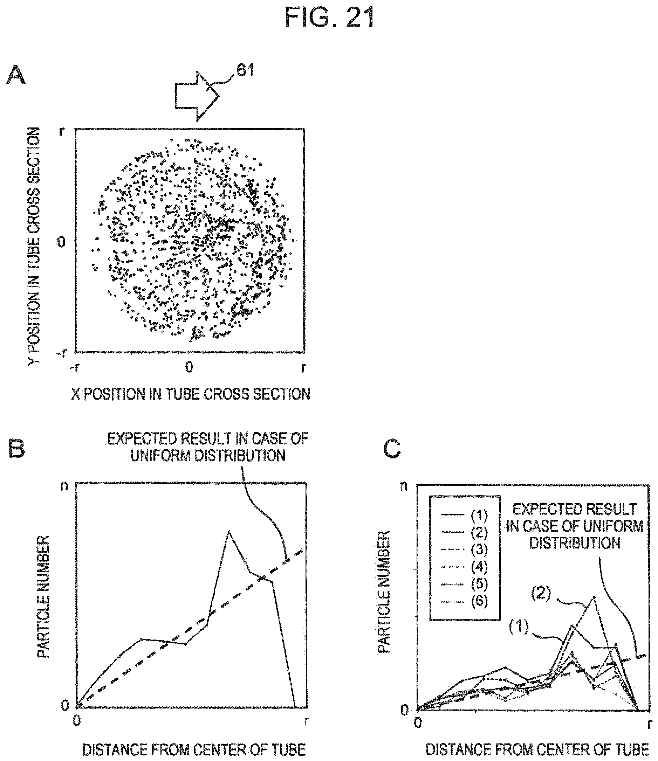

[FIG. 21] A of FIG. 21 is a diagram illustrating a magnetic particle distribution in the cross section 60 in Example 4. B of FIG. 21 is a graph illustrating a relation between a distance from a center and a particle number in the cross section 60 when the axis of the sipper nozzle 30 is the center in Example 4. C of FIG. 21 is a graph illustrating a relation between a distance from the center and a particle number in each divided region in Example 4, similarly to D of FIG. 4.

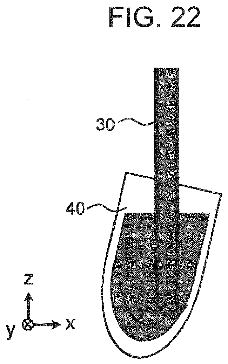

[FIG. 22] FIG. 22 is a diagram illustrating a modification of Example 4, in which the suspension container 40 is provided such that the center axis of the suspension container 40 is inclined in the x axis direction (or a y axis direction) with respect to the center axis of the sipper nozzle 30.

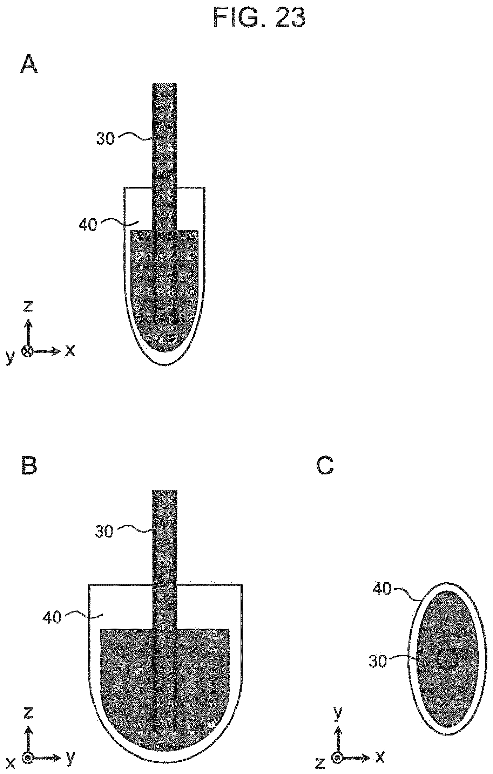

[FIG. 23] A of FIG. 23 is a diagram illustrating a configuration example according to Example 5, which is a cross-sectional configuration example taken along a plane that passes through a center axis of a suspension container 40 and is parallel to a zx plane. B of FIG. 23 is a diagram illustrating a configuration example according to Example 5, which is a cross-sectional configuration example taken along a plane that passes through the center axis of the suspension container 40 and is parallel to a yz plane. C of FIG. 23 is a diagram illustrating a cross-sectional configuration taken along a plane parallel to an xy plane in Example 5.

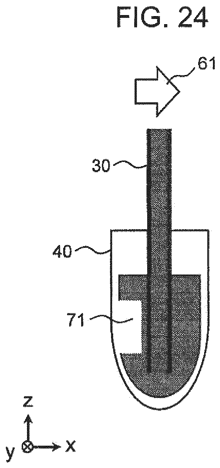

[FIG. 24] FIG. 24 is a diagram illustrating a modification of Example 5, in which a protrusion is provided inside the suspension container 40 in an x axis direction to break axial symmetry of a flow of a fluid (achieve axial asymmetry).

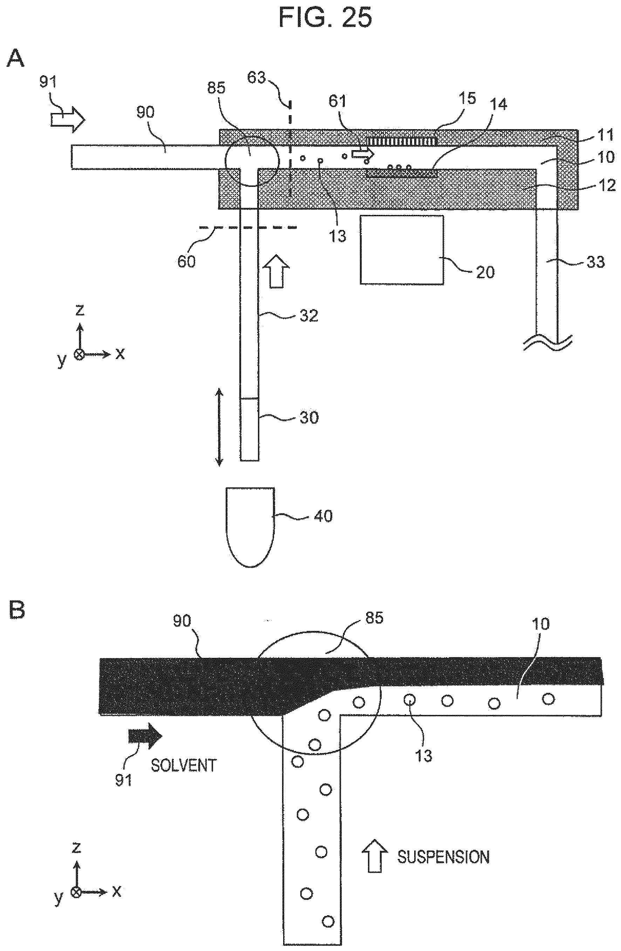

[FIG. 25] A of FIG. 25 is a diagram illustrating a flow channel configuration example according to Example 6, in which a flow channel 90 is connected to an upstream side of the flow channel 10 in a horizontal direction, and is connected to a tube (flow channel) 32 connected downward in a vertical direction (-z direction) at a joining portion 85. B of FIG. 25 is a diagram illustrating a state in which a suspension containing magnetic particles flowing in from the tube (flow channel) 32 and a solvent flowing in from the flow channel 90 are joined at the joining portion 85 to form a parallel flow in the flow channel 10.

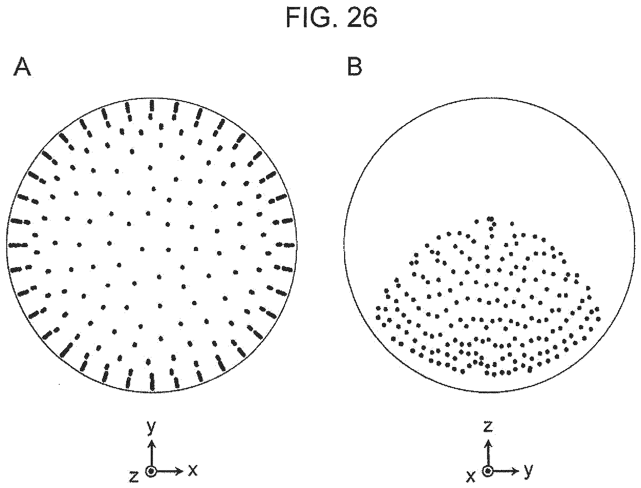

[FIG. 26] A of FIG. 26 is a diagram illustrating a magnetic particle distribution in the cross section 60 in Example 6. B of FIG. 26 is a diagram illustrating a magnetic particle distribution in a cross section 63 in Example 6.

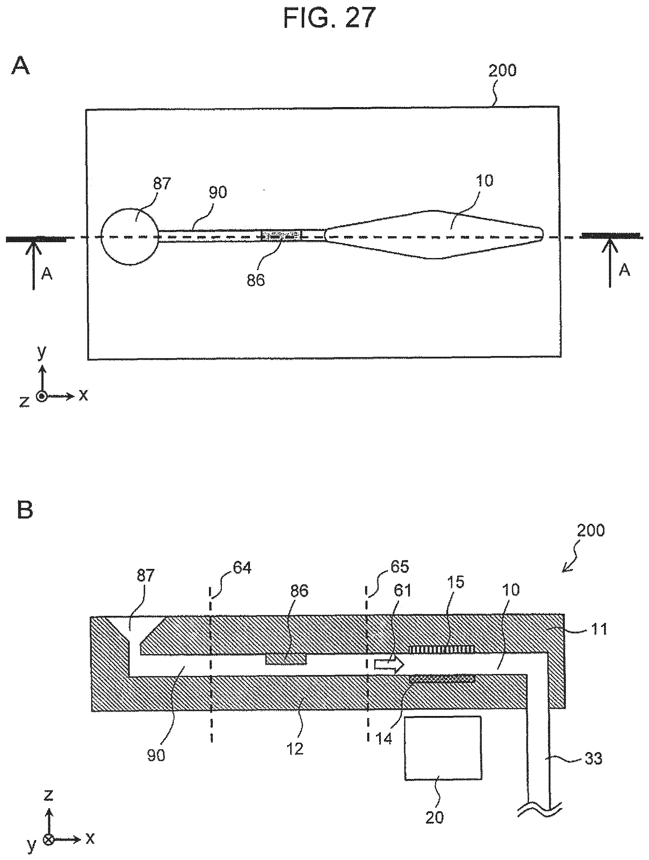

[FIG. 27] A of FIG. 27 is a diagram illustrating an upper surface configuration example of a flow cell 200 according to Example 7. B of FIG. 27 is a diagram illustrating a configuration example taken along an A-A cross section in A of FIG. 27.

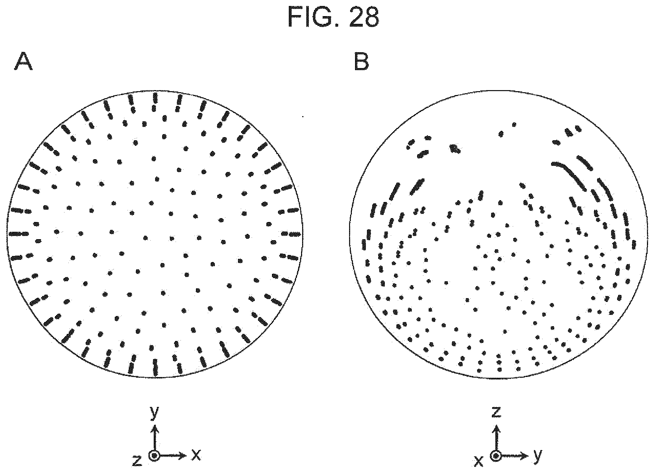

[FIG. 28] A of FIG. 28 is a diagram illustrating a state of a magnetic particle distribution in a cross section 64 on an upstream side of a flow contraction plate 86 in Example 7, in which magnetic particles are uniformly distributed in the cross section 64. B of FIG. 28 is a diagram illustrating a magnetic particle distribution in a cross section 65 on a downstream side of the flow contraction plate 86.

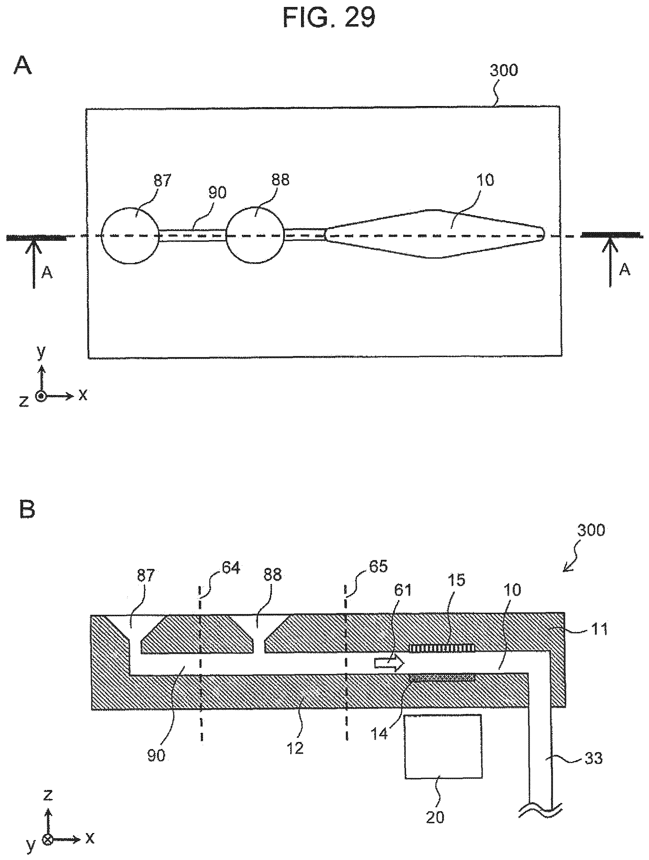

[FIG. 29] A of FIG. 29 is a diagram illustrating an upper surface configuration example of a flow cell 300 according to Example 8. B of FIG. 29 is a diagram illustrating a configuration example taken along an A-A cross section in A of FIG. 29.

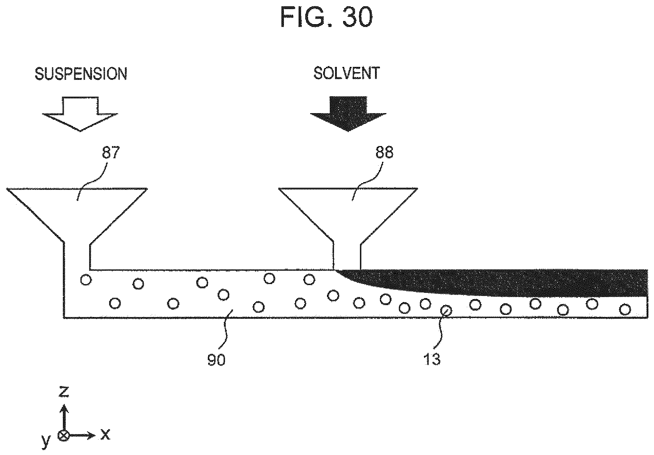

[FIG. 30] FIG. 30 is a diagram illustrating a state of a flow channel when a suspension and a solvent are injected.

### Description of Embodiments

An embodiment of the disclosure relates to a sample analyzer that analyzes a sample, and in particular to a sample analyzer that utilizes a reaction between an antigen and an antibody.

Hereinafter, each example of the embodiment of the disclosure will be described with reference to the accompanying drawings. In the following examples, the same components are denoted by the same reference numerals, and the description thereof may be omitted.

### (1) Example 1

### <Configuration Example of Sample Analyzer>

As an example of a sample analyzer according to the present embodiment, an immunoanalyzer will be described. The present embodiment is not limited to immunoanalysis, and can be applied to any sample analyzer that captures magnetic particles by switching a magnetic field strength using the magnetic particles, and can be similarly used for an analyzer for DNA, biochemistry, or the like. The embodiments described below are not limited to shapes, dimensions, numbers, and the like, and can be changed without departing from the gist thereof, and a plurality of embodiments, including the embodiments described below, may be combined.

FIG. 1 is a diagram illustrating a schematic configuration example of an immunoanalyzer 100 according to the present embodiment. In the immunoanalyzer 100, a portion that includes a flow channel 10, a flow channel top surface wall 11, a flow channel bottom surface wall 12, a reaction field electrode 14, and a counter electrode 15 is referred to as a detection unit (or a flow cell as an integrated member). The flow channel top surface wall 11 of the flow cell is preferably made of a transparent material, for example, glass, plastic, or the like such that a photodetector 23 can detect a wavelength of a light emitted by a labeled substance bound to magnetic particles 13 around the reaction field electrode 14.

The reaction field electrode 14 is provided around a lower peripheral portion of the flow channel 10, and the counter electrode 15 is provided around a peripheral portion facing the reaction field electrode 14 across the flow channel 10. The reaction field electrode 14 and the counter electrode 15 are connected to a voltage application unit 16 via signal lines 55b and 55c. The voltage application unit 16 is connected to a controller 50 by a signal line 55a.

The reaction field electrode 14 and the counter electrode 15 are made of materials, for example, gold, platinum, palladium, tungsten, iridium, nickel, an alloy thereof, or a carbon material. The reaction field electrode 14 and the counter electrode 15 may also be formed using a film formed by plating or sputtering the above material onto a base material such as titanium.

The reaction field electrode 14 basically has a surface shape from a viewpoint of ease of light emission measurement, and is provided on a bottom surface of the flow channel 10 here. However, the reaction field electrode 14 may be on another surface within the flow channel 10, or on a plurality of surfaces three-dimensionally disposed. As long as the reaction field electrode 14 is suitable for capturing the magnetic particles 13 or for the electrochemiluminescence of the labeled substance bound to the magnetic particles 13, it does not necessarily have to be in a surface shape, and may be in a linear shape or a combination of a linear shape and a surface shape. The counter electrode 15 may have any configuration or shape, such as a linear shape, a surface shape, or a combination thereof, as long as it is capable of generating a voltage within the flow channel 10 in combination with the reaction field electrode 14.

To adsorb the magnetic particles, a magnet 20 (permanent magnet or electromagnet) is used as a magnetic field application unit.

The voltage application unit 16 is controlled by the controller 50. When a voltage is applied between the reaction field electrode 14 and the counter electrode 15 in the flow channel 10 by a command from the controller 50 to the voltage application unit 16, the labeled substance bonded to the magnetic particles 13 adsorbed on the reaction field electrode 14 can be electrochemically caused to emit light.

The photodetector 23 can detect a wavelength of light emitted by the labeled substance bound to the magnetic particles 13 around the reaction field electrode 14, and can be, for example, a camera, a photomultiplier, or the like. The photodetector 23 is operated by a signal from the controller 50.

The flow channel 10 in the flow cell is connected to a sipper nozzle 30 and a pump 35 through a tube (flow channel) 32 and a tube 33. The sipper nozzle 30 is movable by an arm 31, and a suspension container 40, a probe cleaner container 42, and a buffer solution container 43 are provided in a movement range thereof.

A valve 36 is provided in the tube 33 between the flow channel 10 and the pump 35. The pump 35 and the valve 36 are connected to the controller 50 through signal lines 52 and 53. Further, the pump 35 and the valve 36 are connected to a waste liquid container 45 through a valve 37 and a tube 34.

The controller 50 is connected to the valves 36 and 37, the pump 35, the arm 31, the voltage application unit 16, a voltage application unit 17, and the photodetector 23 by independent signal lines 53, 51, 52, 54, 55a, 56a, and 57, respectively. Accordingly, each connection portion can be independently controlled.

A sample to be analyzed is a substance derived from a living body such as serum or urine. When the sample is serum, a specific component to be analyzed is, for example, a tumor marker, an antibody, an antigen-antibody complex, or a single protein. In the following description, the specific component is thyroid stimulating hormone (TSH).

The suspension container 40 contains a sample to be analyzed which is mixed with a bead solution and a reagent and then reacted at a certain temperature (for example, 37°C) for a certain time as a pretreatment process. The bead solution is a solution in which the magnetic particles 13 obtained by embedding a particulate magnetic substance in a matrix material such as polystyrene are dispersed in a buffer solution, and streptavidin capable of binding to biotin is bonded to a surface of the matrix material. The reagent contains a substance that binds the magnetic particles 13 to the specific component TSH in the sample, and this substance contains an anti-thyroid stimulating hormone (TSH) antibody having a biotin-treated terminal. The reagent varies depending on a type of the specific component to be analyzed, and may be, for example, an immunoglobulin, an antigen, an antibody, or other biological substances.

The probe cleaner container 42 contains a probe cleaner for cleaning insides of the flow channel 10 and the tube (flow channel) 32.

The flow channel 10 has a shape that is preferably formed such that, when a length (path length) in a direction along a flow and a thickness (vertical direction) and a width (horizontal direction) of a cross section perpendicular to the flow are defined, the path length is 2 to 20 times the greater of the thickness and the width. This is because, by securing a sufficient path length, the magnetic particles 13 in a fluid spread in the flow channel 10, and then are likely to be adsorbed onto the reaction field electrode 14 provided on the bottom surface of the flow channel 10.

An adsorption distribution of the magnetic particles 13 in the flow channel 10 is determined by a magnetic force received from a magnet (permanent magnet or electromagnet) provided in a vicinity of the flow channel 10 and a drag force due to the flow of the fluid. A magnetic field within the flow channel 10 preferably has a magnetic flux density strength of about 0.1 to 0.5 T. A flow rate of the fluid at that time is preferably about 0.05 to 0.10 m/s.

The particles used as the magnetic particles 13 are preferably particles as illustrated below. (i) Particles that exhibit paramagnetism, superparamagnetism, ferromagnetism, or ferrimagnetism, and (ii) particles that exhibit paramagnetism, superparamagnetism, ferromagnetism, or ferrimagnetism, encapsulated in a material such as a synthetic high polymer compound (polystyrene, nylon, or the like), a natural polymer (cellulose, agarose, or the like), or an inorganic compound (silica, or the like). A particle diameter of the magnetic particle is preferably in a range of 0.01 to 200 µm, more preferably in a range of 1 to 10 µm. A specific gravity is preferably 1.3 to 1.5. According to this specification, the magnetic particles 13 are unlikely to settle in the liquid and are likely to be suspended. A substance having a property of specifically binding to a substance to be analyzed, for example, an antibody having a property of specifically binding to an antigen is bound to a surface of the particle.

The labeled substance is preferably a substance as illustrated below. Specifically, from a viewpoint of specifically binding the labeled substance to the substance to be analyzed by an appropriate unit and causing the labeled substance to emit light by an appropriate unit, the following examples can be given.
(a) A labeled substance used in a fluorescent immunoassay method, for example, an antibody labeled with fluorescein isothiocyanate.
(b) A labeled substance used in a chemiluminescent immunoassay method, for example, an antibody labeled with acridinium ester.
(c) A labeled substance used in a chemiluminescent enzyme immunoassay method, for example, an antibody labeled with a chemiluminescent enzyme using luminol or an adamantyl derivative as a luminescent substrate.

The above is the configuration example of the immunoanalyzer 100 (FIG. 1). Next, an operation example of the present embodiment will be described.

### <Operation Example of Immunoanalyzer 100>

In the immunoanalyzer 100, an operation is such that one analysis is defined as one cycle, and a basic device operation state is to continuously perform the cycle a plurality of times.

One analysis cycle includes a suspension aspiration period, a magnetic particle capture period, a detection period, a cleaning period, a reset period, and a preliminary aspiration period. One cycle is started when the suspension container 40 containing the suspension treated in a reaction unit 41 is set at a predetermined position.

In the suspension aspiration period, the valve 36 is set to an open state, and the valve 37 is set to a closed state. The arm 31 is operated in response to a signal from the controller 50 to insert the sipper nozzle 30 into the suspension container 40. Subsequently, in response to a signal from the controller 50, the pump 35 performs a certain amount of aspiration operation, and the suspension in the suspension container 40 enters the tube (flow channel) 32 via the sipper nozzle 30. In this state, the pump 35 is stopped, and the arm 31 is operated to insert the sipper nozzle 30 into a cleaning mechanism 44. When passing through the cleaning mechanism 44, the sipper nozzle 30 (distal end portion) is cleaned.

In a magnetic particle adsorption period, a slide mechanism (not illustrated) is operated in response to a signal from the controller 50, and the magnet 20 moves to a position below the flow channel 10. The pump 35 performs aspiration at a certain speed in response to a signal from the controller 50. Meanwhile, the suspension existing in the tube (flow channel) 32 passes through the flow channel 10. Since a magnetic field is generated from the magnet 20 in flow channel walls 11 and 12, the magnetic particles 13 contained in the suspension are attracted toward the magnet 20 by a magnetic force and captured (adsorbed) by the reaction field electrode 14 at an adsorption predetermined position. After a certain time has elapsed, the aspiration of the suspension by the pump 35 is stopped.

In the detection period, the slide mechanism (not illustrated) is operated to move the magnet 20 away from the flow channel 10. Subsequently, in response to a signal from the controller 50, the photodetector 23 is operated, and a voltage is applied to the reaction field electrode 14 and the counter electrode 15. Accordingly, the light emitted from the labeled substance bound to the magnetic particles 13 can be received by the photodetector 23 while the magnetic particles 13 are held on the reaction field electrode 14, and the measurement can be performed. An intensity of the detected light emission is collected as a signal by the controller 50. After a certain time has elapsed, the application of the voltage to the reaction field electrode 14 and the counter electrode 15 and the photodetector 23 are stopped. During the detection period, the arm 31 is operated to insert the sipper nozzle 30 into the cleaning mechanism 44.

In the cleaning period, the controller 50 uses the pump 35 to aspirate the probe cleaner from the probe cleaner container 42 and causes the aspirated probe cleaner to pass through the flow channel 10. At this time, since the magnet 20 is away from the flow channel 10, the magnetic particles 13 are not held on the reaction field electrode 14 and are washed away together with the probe cleaner.

In the reset period, the controller 50 closes the valve 36, opens the valve 37, and causes the pump 35 to perform a discharge operation. The liquid in the pump 35 is discharged to the waste liquid container 45.

In the preliminary aspiration period, the controller 50 causes the pump 35 to operate to aspirate the buffer solution from the buffer solution container 43, and fills the tube (flow channel) 32 and the flow channel 10 with the buffer solution. After the preliminary aspiration period, a next cycle is executable.

### <Considerations on Relation Between Magnetic Particle Distribution in Sipper Nozzle 30 and Captured Magnetic Particle Distribution>

### (i) Case of Existing Configuration Example

With reference to FIGS. 2 to 6, an example in which a relation between a magnetic particle distribution in a cross section 60 of the sipper nozzle 30 and a magnetic particle distribution captured on the reaction field electrode 14 is calculated by a fluid and magnetic field analysis in a sample analyzer (immunoanalyzer) according to an existing configuration example will be described.

FIG. 2 is a diagram illustrating a flow cell and a magnetic field structure according to the existing configuration example using a permanent magnet. FIG. 2 illustrates a state of a cross section of a zx plane in a case in which a coordinate system, in which a flow direction 61 in the flow channel 10 is an x axis, a horizontal direction of a cross section perpendicular to the flow is a y axis, and the vertical direction is a z axis, is placed in a vicinity of a flow cell of an existing immunoanalyzer. A vicinity of the flow channel 10 includes the flow channel 10, the flow channel top surface wall 11, the flow channel bottom surface wall 12, the magnetic particles 13, the reaction field electrode 14, the counter electrode 15, and the magnet 20. The magnetic particles 13 flow in the flow channel 10 along the flow direction 61. The tube (flow channel) 32 is connected downward in the vertical direction at a bent portion 80.

FIG. 3 is a diagram illustrating a state when the sipper nozzle 30 is inserted into the suspension container 40 and the suspension is aspirated in the suspension aspiration period. A of FIG. 3 illustrates a position relation between the sipper nozzle 30 and the suspension container 40. B of FIG. 3 illustrates a fluid region for which a calculation is performed in fluid analysis. C of FIG. 3 illustrates a cross section taken along a plane that passes through a center axis of the sipper nozzle 30 and is parallel to the zx plane in a sipper nozzle distal end portion 38. D of FIG. 3 illustrates a cross section taken along a plane that passes through the center axis of the sipper nozzle 30 and is parallel to a yz plane. E of FIG. 3 illustrates a cross section taken along a plane parallel to an xy plane of the sipper nozzle 30. In the existing configuration example, the sipper nozzle 30 is disposed to include a center axis of the suspension container 40, and is disposed in a direction substantially perpendicular to the flow direction 61 in the flow channel 10. The sipper nozzle 30 has a cylindrical shape.

FIG. 4 illustrates a fluid analysis result obtained by calculating the magnetic particle distribution in the cross section 60 in FIG. 2 when the suspension is aspirated via the sipper nozzle 30 from the state in which the magnetic particles 13 are uniformly dispersed in the suspension container 40 in the fluid region in B of FIG. 3. An analysis model is symmetrical with respect to a plane that includes the center axis of the sipper nozzle 30 and is parallel to the zx plane. A of FIG. 4 illustrates the magnetic particle distribution in the cross section 60 in FIG. 2. B of FIG. 4 illustrates a state in which a region in A of FIG. 4 is divided into 12 in a circumferential direction. C of FIG. 4 is a graph illustrating a relation between a distance from a center and a particle number in the cross section 60 when the axis of the sipper nozzle 30 is the center. D of FIG. 4 is a graph illustrating a relation between a distance from a center of the sipper nozzle and a particle number in each region divided in B of FIG. 4. In the existing configuration example, as can be seen from A and C of FIG. 4, in the cross section 60, the particles exhibit higher particle density near the center axis of the sipper nozzle 30 and slightly inward from an outermost periphery. Further, as can be seen from D of FIG. 4, the magnetic particle distribution in the circumferential direction has almost no difference and is axisymmetric.

FIG. 5 illustrates a distribution of the magnetic particles 13 in the cross section 60 of the sipper nozzle 30 based on results in A and C of FIG. 4. When a fluid-magnetic field analysis for capturing the magnetic particles 13 on the reaction field electrode 14 was performed with this state as an initial condition, as illustrated in FIG. 6, it was found that only the magnetic particles in the region illustrated in black were captured on the reaction field electrode 14, and the magnetic particles in the region illustrated in gray were not captured on the reaction field electrode 14, passed through the flow channel 10, and flowed out to the tube 33.

A of FIG. 7 illustrates a schematic diagram of the magnetic particle distribution in the cross section 60 in the existing configuration example illustrated in FIGS. 2 to 6. As illustrated in A of FIG. 7, in the existing configuration example, the particle distribution density is high near the center axis and slightly inside the outermost peripheral portion.

### (ii) Case of the Present Embodiment

B and C of FIG. 7 are schematic diagrams of the magnetic particle distribution in the cross section 60 achieved in the present embodiment. In B of FIG. 7, the magnetic particle distribution density is low on an inner side of the cross section 60, and the magnetic particle distribution density is high on an outer side of the cross section 60. In C of FIG. 7, the magnetic particle distribution density is low on an upstream side and the magnetic particle distribution density is high on a downstream side with respect to the flow direction 61 in the flow channel 10. As a result of the above fluid-magnetic field analysis, the particle number captured on the reaction field electrode 14 can be increased in B of FIG. 7 as compared with FIG. 6. Further, in C of FIG. 7, the particle number captured on the reaction field electrode 14 can be further increased. Since a flow velocity is high in a central region of the sipper nozzle 30, when the flow direction changes at the bent portion 80, the magnetic particles 13 distributed in the central region move to an upper surface side of the flow channel 10 by a centrifugal force. Therefore, in the cross section 60 on the upstream side, it is important to distribute a large amount of the magnetic particles 13 not in the central region of the sipper nozzle 30 but on an outer peripheral side. Further, in a case of bending in a +x direction in the bent portion 80 as in FIG. 2, the particles present on a +x side in the cross section 60 flow on an inner peripheral side than the particles present on a -x side, and as a result, the particles flow in a vicinity of the reaction field electrode and are easily captured. Therefore, it is more desirable that the magnetic particles 13 be more densely distributed on the outer peripheral side and the +x side, as illustrated in C of FIG. 7. However, it is important to distribute the magnetic particles 13 on the outer peripheral side on the premise that the bent portion 80 is provided.

Further, FIG. 8 is a diagram illustrating a fluid analysis result that explains a relation between magnetic particle distributions in the cross section 60 on the upstream side and in the cross section 63 on the downstream side of the bent portion 80 in FIG. 2. A of FIG. 8 illustrates an initial state of the analysis, in which magnetic particles are distributed outside the flow channel in the cross section 60. B of FIG. 8 illustrates the magnetic particle distribution in the cross section 63 after passing through the bent portion 80 from the initial state in A of FIG. 8. On the other hand, C of FIG. 8 illustrates a state in which magnetic particles are distributed on the inner side of the flow channel in the cross section 60. D of FIG. 8 illustrates the magnetic particle distribution in the cross section 63 after passing through the bent portion 80 from the initial state in C of FIG. 8. From the result in B of FIG. 8, it can be seen that when a magnetic particle distribution density is high on an outer side in the cross section 60 on the upstream side of the bent portion 80, the particles are biased in a -z direction of the flow channel in the cross section 63 on the downstream side of the bent portion 80. On the other hand, from the result in D of FIG. 8, it can be seen that when a magnetic particle distribution density is high on an inner side (central region) in the cross section 60 on the upstream side of the bent portion 80, the magnetic particles are biased in a +z direction in the cross section 63 on the downstream side of the bent portion 80.

Therefore, to increase the magnetic particle number captured on the reaction field electrode 14, it is important to distribute the magnetic particles such that the magnetic particle distribution density is high on the same side as the magnet 20 in the flow channel cross section 63 on the downstream side of the bent portion 80 as illustrated in B of FIG. 8. When such a magnetic particle distribution is achieved in the cross section 63, the flow channel 10 is more easily affected by the magnetic field of the magnet 20, and the number of captured magnetic particles can be increased.

To realize the magnetic particle distribution in the cross section 63 as illustrated in B of FIG. 8, it is important to distribute the magnetic particles such that the magnetic particle distribution density is low on the inner side and the magnetic particle distribution density is high on the outer side in the flow channel cross section 60 on the upstream side of the bent portion 80 as illustrated in B and C of FIG. 7.

### <Configuration Example of Sipper Nozzle 30>

FIG. 9 is a diagram illustrating a configuration example (shape example) of the sipper nozzle 30 according to Example 1. A of FIG. 9 illustrates a position relation between the sipper nozzle 30 and a suspension container 40. B of FIG. 9 is a diagram illustrating a cross-sectional configuration taken along a plane that passes through a center axis of the sipper nozzle 30 and is parallel to a zx plane in a distal end portion 38 of the sipper nozzle 30. C of FIG. 9 is a diagram illustrating a cross-sectional configuration taken along a plane that passes through the center axis of the sipper nozzle 30 and is parallel to a yz plane. D of FIG. 9 is a diagram illustrating a cross-sectional configuration taken along a plane parallel to an xy plane of the sipper nozzle 30. The sipper nozzle 30 in Example 1 has an injection needle shape (the distal end portion 38 is obliquely cut, and a shape of an opening is an ellipse rather than a perfect circle), and is configured such that, for example, a length on the upstream side is short and a length on the downstream side is long with respect to the flow direction 61 in the flow channel 10. In B of FIG. 9, an angle formed by a cut surface at a distal end of the sipper nozzle 30 and a horizontal plane is α.

FIG. 10 illustrates a fluid analysis result of a magnetic particle distribution in the cross section 60 of the sipper nozzle 30 when the suspension is aspirated via the sipper nozzle 30 from the state in which the magnetic particles 13 are uniformly dispersed in the suspension container 40 using the injection needle-shaped sipper nozzle 30 according to Example 1 illustrated in FIG. 9. A of FIG. 10 illustrates the magnetic particle distribution in the cross section 60. B of FIG. 10 is a graph illustrating a relation between a distance from a center and a particle number in the cross section 60 when the axis of the sipper nozzle 30 is the center. C of FIG. 10 is a graph illustrating a relation between a distance from the center and a particle number in each region divided in a circumferential direction as in D of FIG. 4. From A and B of FIG. 10, it can be seen that when the sipper nozzle 30 according to Example 1 is used, the particle distribution in cross section 60 of the sipper nozzle 30 is such that the particle number density on an inner side of the cross section (near the central axis) decreases and the particle number density on an outer side of the cross section increases, compared with the case of the existing configuration example illustrated in A and C of FIG. 4. Further, from A and C of FIG. 10, it can be seen that the particle number density is higher on the downstream side than that on the upstream side with respect to the flow direction 61 in the flow channel 10. Therefore, when the sipper nozzle 30 according to Example 1 is used, the magnetic particle distribution approaches that illustrated in C of FIG. 7, and the magnetic particle number captured on the reaction field electrode 14 can be increased.

### <Relation Between Angle α and Magnetic Particle Distribution >

FIG. 11 is a diagram illustrating a fluid analysis result of the magnetic particle distribution in the cross section 60 when the angle α is changed. The hatched region in A of FIG. 11 is defined as Area A. B of FIG. 11 is a diagram illustrating a ratio of particles distributed in AREA A when the angle α is changed. C to E of FIG. 11 illustrate the particle distribution in the cross section 60.

Referring to C to E of FIG. 11, it can be seen that the particle number on the inner side of the cross section 60 decreases and the particle number on the outer side of the cross section 60 increases as the angle α increases. Further, from B of FIG. 11, it can be seen that when the angle α is 20° or more, the particle number distributed in Area A increases, and when the angle α is more than 60°, the particle number distributed in Area A further increases, that is, the particle number captured on the reaction field electrode 14 increases.

In Example 1, the sipper nozzle 30 is described as being short on the upstream side and long on the downstream side with respect to the flow direction 61 in the flow channel 10. However, the same effect can be expected as long as the sipper nozzle 30 has an asymmetric shape with respect to any plane (plane forming any angle with the flow direction 61) passing through the center axis of the nozzle (axis in a direction at which the fluid flows). For example, the sipper nozzle 30 may be formed such that the length on the upstream side is long and the length on the downstream side is short at the distal end portion of the sipper nozzle 30. In this case, the particle distribution illustrated in A of FIG. 10 is inverted with respect to the x direction, and the particle number density is higher on the upstream side than that on the downstream side with respect to the flow direction 61 in the flow channel 10. However, since the particle number density on the inner side of the cross section decreases and the particle number density on the outer side of the cross section increases, the magnetic particle number captured on the reaction field electrode 14 can be increased.

### <Modifications>

FIG. 12 is a diagram illustrating a configuration example of Modification 1 of Example 1. FIG. 13 is a diagram illustrating a configuration example of Modification 2 of Example 1. Modifications 1 and 2 are examples in which the distal end portion of the sipper nozzle 30 is processed into a shape different from that in FIG. 9. A of FIG. 12 and A of FIG. 13 are diagrams each illustrating a position relation between the sipper nozzle 30 and the suspension container 40. B of FIG. 12 and B of FIG. 13 are diagrams each illustrating a cross section taken along a plane that passes through a center axis of the sipper nozzle 30 and is parallel to a zx plane in the cross section 60. C of FIG. 12 and C of FIG. 13 are diagrams each illustrating a cross section taken along a plane that passes through the center axis of the sipper nozzle 30 and is parallel to a yz plane. D of FIG. 12 and D of FIG. 13 are diagrams each illustrating a cross section taken along a plane parallel to an xy plane of the sipper nozzle 30.

A distal end shape of the sipper nozzle 30 illustrated in FIG. 12 is formed into a stepped shape that is short on an upstream side and long on a downstream side with respect to the flow direction 61 in the flow channel 10. A distal end shape of the sipper nozzle 30 illustrated in FIG. 13 is processed on both the upstream side and the downstream side to be short on the upstream side and long on the downstream side with respect to the flow direction 61 in the flow channel 10.

As in the modifications, a flow channel from the suspension container 40 to the flow channel 10, that is, a flow channel structure in the sipper nozzle 30 and the tube (flow channel) 32 is asymmetric with respect to the flow direction 61 in the flow channel 10. Accordingly, as described above, a state in which the magnetic particle distribution density in the cross section 60 is low on an inner side and high on an outer side of the cross section, and is low on the upstream side and high on the downstream side with respect to the flow direction 61 in the flow channel 10, can be created. Therefore, the magnetic particle number captured on the reaction field electrode 14 can be increased. Similarly to the configuration illustrated in FIG. 9, the sipper nozzles 30 according to modifications in FIGS. 12 and 13 may have asymmetry with respect to any plane passing through the center axis.

The configuration described in Example 1 is an example, and as described above, a configuration in which each component is inverted in the X direction may also be used. Even in such a configuration, since axial symmetry with respect to the axial direction of the sipper nozzle 30 is broken, and the particle number density on the inner side of the cross section decreases and the particle number density on the outer side of the cross section increases in the particle distribution on the cross section 60, the magnetic particle number captured on the reaction field electrode 14 can be increased.

### (2) Example 2

(i) FIG. 14 is a diagram illustrating a position relation between the sipper nozzle 30 according to Example 2 and the suspension container 40. The sipper nozzle 30 according to Example 2 has at least two bent portions in the flow channel between a distal end of the sipper nozzle 30 and the bent portion 80 in FIG. 2.

FIG. 15 is a diagram illustrating a fluid analysis result in Example 2. A of FIG. 15 illustrates a magnetic particle distribution in the cross section 60. B of FIG. 15 is a graph illustrating a relation between a distance from a center and a particle number in the cross section 60 when an axis of the sipper nozzle 30 is the center. Further, similarly to D of FIG. 4, C of FIG. 15 is a graph illustrating a relation between a distance from the center of the sipper nozzle 30 and a particle number in each divided region.

Referring to A and B of FIG. 15, it can be seen that when the suspension is aspirated using the sipper nozzle 30 according to Example 2, the magnetic particle distribution in the cross section 60 of the sipper nozzle 30 is such that the particle number density on the inner side of the cross section (near the central axis) decreases and the particle number density on the outer side of the cross section increases, compared with the case of the existing configuration example illustrated in A and C of FIG. 4. Referring to A and C of FIG. 15, it can be seen that the particle number density on the downstream side is higher than that on the upstream side with respect to the flow direction 61 in the flow channel 10.

Therefore, with the configuration of the sipper nozzle 30 according to Example 2, the magnetic particle number captured on the reaction field electrode 14 can be increased. In Example 2, a case in which the sipper nozzle 30 has two bent portions in the flow channel between the distal end of the sipper nozzle 30 and the bent portion 80 in FIG. 2 has been described. However, the number of bent portions may not be two and may be one or three or more. Further, an example in which the flow channel is bent at 90 degrees is illustrated in Example 2. However, the flow channel may be bent at other angles or may have a smoothly curved flow channel shape. Furthermore, the bent portions provided at a plurality of positions in the sipper nozzle 30 according to Example 2 may not be configured to be bent in the flow direction 61 in the flow channel 10, and the bent portions may be provided to have asymmetry with respect to any plane passing through the center axis as in the configuration illustrated in FIG. 9.

(ii) The configuration described in Example 2 is an example, and a configuration in which each component is inverted in the X direction may also be used. Even in such a configuration, since the axial symmetry with respect to the axial direction of the sipper nozzle 30 is broken, and the particle number density on the inner side of the cross section decreases and the particle number density on the outer side of the cross section increases in the particle distribution on the cross section 60, the magnetic particle number captured on the reaction field electrode 14 can be increased.

### (3) Example 3

Example 3 proposes a configuration in which a flow channel structure asymmetric with respect to the flow direction 61 in the flow channel 10 is formed by processing an intermediate flow channel of the sipper nozzle 30. Example 3 will be described with reference to FIGS. 16 to 19.

(i) In the sipper nozzle 30 illustrated in FIG. 16, a flow contraction portion 82 is provided midway along the flow channel such that the flow channel narrows in the x direction.

Further, in the sipper nozzle 30 illustrated in FIG. 17, a hole 83 is formed in the x direction on a side surface midway along the flow channel. In this case, the suspension is aspirated from two directions, that is, the distal end of the sipper nozzle 30 and the hole 83.

In the sipper nozzle 30 illustrated in FIG. 18, a branch flow channel 84 is connected to the side surface midway along the flow channel in the x direction. Also in this case, the suspension is aspirated from two directions, that is, the distal end of the sipper nozzle 30 and the branch flow channel 84.

In the sipper nozzle 30 illustrated in FIG. 19, the sipper nozzle 30 is formed such that a cross-sectional area in a plane parallel to the xy plane decreases from upstream to downstream, and a flow channel center in each cross section moves in the +x direction from upstream to downstream.

With these configurations, a flow channel structure asymmetric with respect to the flow direction 61 in the flow channel 10 can be formed. Therefore, the magnetic particle distribution in the sipper nozzle 30 can be made sparse on the inner side of the tube cross section and dense on the outer side of the tube cross section, and the magnetic particle number captured on the reaction field electrode 14 can be increased. Similarly to the configuration illustrated in FIG. 9, the sipper nozzle 30 according to Example 3 may also have asymmetry with respect to any plane passing through the center axis. Therefore, the hole 83 (FIG. 17) and the branch flow channel 84 (FIG. 18) can be provided at any position of the sipper nozzle 30. Further, a direction in which the flow channel center moves (in FIG. 19) can be any direction in the sipper nozzle 30 (for example, a y direction or a direction forming 45 degrees with the x axis).

(ii) The configuration described in Example 3 is an example, and a configuration in which each component is inverted in the X direction may also be used. Even in such a configuration, since the axial symmetry with respect to the axial direction of the sipper nozzle 30 is broken, and the particle number density on the inner side of the cross section decreases and the particle number density on the outer side of the cross section increases in the particle distribution on the cross section 60, the magnetic particle number captured on the reaction field electrode 14 can be increased.

### (4) Example 4

Example 4 relates to a mode in which the magnetic particle distributions in B and C of FIG. 7 and A and B of FIG. 8 are achieved by controlling provision positions of the sipper nozzle 30 and the suspension container 40. Specifically, Example 4 proposes that the flow of the suspension is biased by offsetting an insertion position of the sipper nozzle 30 into the suspension container 40 from a container center axis and making a fluid region in the suspension container 40 asymmetric with respect to the center axis of the sipper nozzle 30.

### (i) Basic Configuration Example

FIG. 20 is a diagram illustrating a state in which the center axis of the sipper nozzle 30 is disposed offset in the x axis direction with respect to the center axis of the suspension container 40. FIG. 21 is a diagram illustrating a fluid analysis result in a disposition relation illustrated in FIG. 20. A of FIG. 21 illustrates a magnetic particle distribution in the cross section 60. B of FIG. 21 is a graph illustrating a relation between a distance from a center and a particle number in the cross section 60 when the axis of the sipper nozzle 30 is the center. C of FIG. 21 is a graph illustrating a relation between a distance from the center and a particle number in each divided region, similarly to D of FIG. 4.

According to Example 4, for example, in response to an instruction from an operator or in response to a preset command, the controller 50 controls a movement of the sipper nozzle 30, and controls the position relation between the sipper nozzle 30 and the suspension container 40 such that the sipper nozzle 30 is inserted at a position shifted from the axial center of the suspension container 40 (for example, an offset amount of 0.5 mm to 3 mm). Then, the controller 50 causes the pump 35 to operate in a state in which the sipper nozzle 30 is inserted into the suspension container 40 (FIG. 20) to aspirate the suspension. Then, in the magnetic particle distribution in the cross section 60 of the sipper nozzle 30, the particle number density on the inner side of the cross section (near the center axis (center)) decreases and the particle number density on the outer side of the cross section increases, compared with the case of the existing configuration example illustrated in A and B of FIG. 4. As illustrated in A and B of FIG. 21, the particle number density on the downstream side is higher than that on the upstream side with respect to the flow direction 61 in the flow channel 10. Therefore, the magnetic particle number captured on the reaction field electrode 14 can be increased by controlling the insertion position of the sipper nozzle 30 according to Example 4 into the suspension container 40.

### (ii) Modification

FIG. 22 is a diagram illustrating a modification of Example 4, in which the suspension container 40 is provided such that the center axis of the suspension container 40 is inclined in the x axis direction (or the y axis direction) with respect to the center axis of the sipper nozzle 30.

According to this modification, similarly to the basic configuration example, a fluid region formed by the flow channel in the suspension container 40 and the sipper nozzle 30 can be asymmetrically formed with respect to the flow direction 61 in the flow channel 10. Therefore, the particle number density on the inner side of the cross section (near the center axis) decreases, and the particle number density on the outer side of the cross section increases. Further, the particle number density is higher on the downstream side than that on the upstream side with respect to the flow direction 61 in the flow channel 10, and the magnetic particle number captured on the reaction field electrode 14 can be increased.

An inclination of the suspension container 40 can be achieved, for example, by controlling, by the controller 50, an inclination of a stage (not illustrated) on which the suspension container 40 is fixedly placed, or by configuring the suspension container 40 to have an inclination from the beginning in a state of being placed on the stage.

(iii) The configuration described in Example 4 is an example, and a configuration in which each component is inverted in the X direction may also be used. Even in such a configuration, since the axial symmetry with respect to the axial direction of the sipper nozzle 30 is broken, and the particle number density on the inner side of the cross section decreases and the particle number density on the outer side of the cross section increases in the particle distribution on the cross section 60, the magnetic particle number captured on the reaction field electrode 14 can be increased.

### (5) Example 5

Example 5 relates to a mode in which the magnetic particle distributions in B and C of FIG. 7 and A and B of FIG. 8 are achieved by devising a shape of the suspension container 40.

### (i) Basic Configuration Example

FIG. 23 is a diagram illustrating a configuration example of the suspension container 40 according to Example 5. A of FIG. 23 is a diagram illustrating a cross-sectional configuration taken along a plane that passes through a center axis of the suspension container 40 and is parallel to a zx plane. B of FIG. 23 is a diagram illustrating a cross-sectional configuration taken along a plane that passes through the center axis of the suspension container 40 and is parallel to a yz plane. C of FIG. 23 is a diagram illustrating a cross-sectional configuration taken along a plane parallel to an xy plane. As illustrated in C of FIG. 23, the suspension container 40 illustrated in Example 5 has an elliptical shape in which the x axis direction is a minor axis and the y axis direction is a major axis in the cross section in the plane parallel to the xy plane.

With this configuration, axial symmetry of the flow of the fluid flowing from the suspension container 40 into the sipper nozzle 30 is broken. Therefore, in the cross section 60 of the sipper nozzle 30, the particle number density on the inner side of the cross section (near the center axis) decreases and the particle number density on the outer side of the cross section increases, compared with the existing configuration example described above, and the magnetic particle number captured on the reaction field electrode 14 can be increased.

### (ii) Modification

FIG. 24 is a diagram illustrating a modification of Example 5, in which a protrusion is provided inside the suspension container 40 in the x axis direction to break axial symmetry of the flow of the fluid (achieve axial asymmetry). According to the configuration illustrated in the modification, the axial symmetry of the flow of the fluid flowing from the suspension container 40 into the sipper nozzle 30 is broken by the protrusion. Therefore, in the cross section 60 of the sipper nozzle 30, the particle number density on the inner side of the cross section (near the center axis) decreases and the particle number density on the outer side of the cross section increases, compared with the existing configuration example described above. Accordingly, the magnetic particle number captured on the reaction field electrode 14 can be increased.

(iii) The configuration described in Example 5 is an example, and a configuration in which each component is inverted in the X direction may also be used. Even in such a configuration, since the axial symmetry with respect to the axial direction of the sipper nozzle 30 is broken, and the particle number density on the inner side of the cross section decreases and the particle number density on the outer side of the cross section increases in the particle distribution on the cross section 60, the magnetic particle number captured on the reaction field electrode 14 can be increased.

### (6) Example 6

Example 6 relates to a mode in which a distribution of magnetic particles contained in the suspension aspirated from the sipper nozzle 30 in the flow channel 10 is made as illustrated in B of FIG. 8 by flowing a solvent from a path different from the sipper nozzle 30.

FIG. 25 is a diagram illustrating a flow channel configuration according to Example 6 and a state in which the magnetic particle distribution illustrated in B of FIG. 8 is formed. FIG. 26 illustrates a fluid analysis result. A of FIG. 26 is a diagram illustrating a magnetic particle distribution in the cross section 60. In this analysis, a uniformly dispersed state illustrated in A of FIG. 26 is an initial state. B of FIG. 26 is a diagram illustrating a magnetic particle distribution in the cross section 63.

As illustrated in A of FIG. 25, a flow channel 90 is connected to the upstream side of the flow channel 10 in a horizontal direction, and is connected to the tube (flow channel) 32 connected downward in a vertical direction (-z direction) at a joining portion 85. A mechanism (not illustrated) for flowing a solvent is connected to the flow channel 90. In the flow channel 90, a solvent that does not contain the magnetic particles constituting the suspension flows in a flow direction 91.

According to the configuration of Example 6, as illustrated in B of FIG. 25, the suspension containing magnetic particles flowing in from the tube (flow channel) 32 and the solvent flowing in from the flow channel 90 join at the joining portion 85, and a parallel flow is formed in the flow channel 10. The suspension containing the magnetic particles 13 mainly flows along a lower side of the flow channel 10 in the vertical direction, and the solvent flowing from the flow channel 90 mainly flows along an upper side in the vertical direction. Therefore, as illustrated in B of FIG. 26, the magnetic particles 13 flow along the lower side of the flow channel 10 in the vertical direction. Therefore, it is likely to be affected by the magnetic force of the magnet 20 and is likely to be captured on the reaction field electrode 14. As a result, the number of captured magnetic particles can be increased.

### (7) Example 7

Example 7 relates to Mode 1 in which the magnetic particle distribution illustrated in B of FIG. 8 is achieved in a flow cell 200 that does not include the sipper nozzle 30.

FIG. 27 is a diagram illustrating a configuration example of a flow cell according to Example 7. A of FIG. 27 is a diagram illustrating an upper surface configuration example of the flow cell 200 according to Example 7. B of FIG. 27 is a diagram illustrating a configuration example taken along an A-A cross section in A of FIG. 27. FIG. 28 is a diagram illustrating a fluid analysis result in Example 7. A of FIG. 28 is a diagram illustrating a magnetic particle distribution in a cross section 64 on an upstream side of a flow contraction plate 86, in which magnetic particles are uniformly distributed in the cross section 64. B of FIG. 28 is a diagram illustrating a magnetic particle distribution in a cross section 65 on a downstream side of the flow contraction plate 86.

The flow cell 200 according to Example 7 is configured as a plate-shaped flow cell in which a suspension injection port 87, the flow channel 90, and the flow channel 10 are integrated. In the flow cell 200, the flow channel 90 is connected to an upstream side of the flow channel 10 in a horizontal direction, and the suspension injection port 87 for supplying the suspension is connected to the upstream side thereof. The flow contraction plate 86 is provided midway along the flow channel 90, on an upper side in the vertical direction, that is, on the flow channel top surface wall 11 such that a width of the flow channel 90 is reduced.

A suspension containing magnetic particles prepared in advance is supplied to the suspension injection port 87 by, for example, a nozzle (not illustrated). Then, the pump 35 is operated by the controller 50, and the supplied suspension flows in the flow direction 61 and passes through the flow contraction plate 86. When the magnetic particles 13 uniformly distributed on the upstream side of the flow contraction plate 86 (see A of FIG. 28) pass through the flow contraction plate 86, the distribution of the magnetic particles 13 is biased to a lower side in the vertical direction as illustrated in B of FIG. 28. Therefore, since the magnetic particles 13 are likely to be affected by the magnetic force of the magnet 20 in the flow channel 10, the magnetic particles 13 are likely to be captured on the reaction field electrode 14. As a result, the number of captured magnetic particles 13 can be increased.

In Example 7, a configuration in which the flow contraction plate 86 is provided in the flow channel 90 is illustrated. However, the flow contraction plate 86 may be provided in the flow channel 10 upstream of the reaction field electrode 14, which is a capture region. The closer the position of the flow contraction plate 86 is to the capture region, the higher the capture effect is.

### (8) Example 8

Example 8 relates to Mode 2 in which the magnetic particle distribution illustrated in B of FIG. 8 is achieved in a flow cell 300 that does not include the sipper nozzle 30.

FIG. 29 is a diagram illustrating a configuration example of a flow cell according to Example 8. A of FIG. 29 is a diagram illustrating an upper surface configuration example of the flow cell 300 according to Example 8. B of FIG. 29 is a diagram illustrating a configuration example taken along an A-A cross section in A of FIG. 29. FIG. 30 is a diagram illustrating a state of a flow channel when a suspension and a solvent are injected.

The flow cell 300 according to Example 8 is configured as a plate-shaped flow cell in which the suspension injection port 87, a solvent injection port 88, the flow channel 90, and the flow channel 10 are integrated. In the flow cell 300, the flow channel 90 is connected to an upstream side of the flow channel 10 in a horizontal direction. The suspension injection port 87 and the solvent injection port 88 are provided on an upstream side of the flow channel 90. The suspension injection port 87 is provided upstream of the solvent injection port 88.

A suspension containing magnetic particles prepared in advance is supplied to the suspension injection port 87 by, for example, a nozzle (not illustrated), and a solvent containing no magnetic particles is supplied to the solvent injection port 88. Under the control of the controller 50, the suspension and the solvent supplied by the operation of the pump 35 flow in the flow direction 61. The suspension containing the magnetic particles joins with the solvent when passing through a connection portion of the solvent injection port 88, and generates a parallel flow as illustrated in FIG. 30. At this time, the magnetic particles 13 flow along a lower side of the flow channel 90 and the flow channel 10 in the vertical direction. Therefore, the magnetic particles 13 are likely to be affected by the magnetic force of the magnet 20, and thus are likely to be captured on the reaction field electrode 14. As a result, the number of captured magnetic particles can be increased.

In Example 8, a plate in which the suspension injection port 87 and the solvent injection port 88 are integrated has been described. However, a plate in which, for example, a flow channel, an injection port, or a joining portion for performing a pretreatment to prepare a suspension is provided further upstream may also be used.

### (9) Summary

(i) Embodiments of the disclosure will be described with various examples. Here, expressing to include all examples, a sample analyzer according to the disclosure (immunoanalyzer 100) is characterized by including a magnetic particle distribution adjustment unit (configuration of the magnetic particle distribution adjustment unit differs in Example 1 to Example 8) that makes, for a distribution of magnetic particles 13 in a cross section perpendicular to the flow direction 61 of a sample liquid (suspension) in a capture region (reaction field electrode 14) of the flow channel 10, a distribution in a proximal region closer to the capture region (reaction field electrode 14) than a center axis of the flow channel 10 denser than a distribution in a distal region farther from the central axis as viewed from the capture region. By providing the magnetic particle distribution adjustment unit, in the sample analyzer, capture efficiency of magnetic particles at a predetermined position can be increased, and detection can be performed with high sensitivity.
(ii) According to Example 1, the flow channel 10 including the capture region (reaction field electrode 14) is placed in a horizontal direction. At this time, the magnetic particle distribution adjustment unit includes a sample liquid introduction tube (sipper nozzle 30) extending from the flow channel 10 in a vertical direction. The sample liquid introduction tube (sipper nozzle 30) is configured to have an asymmetrical shape in a predetermined plane including a center axis of the sipper nozzle 30. At this time, when the sample liquid (suspension) is introduced into the flow channel 10 via the sipper nozzle 30, the distribution of the magnetic particles 13 in a flow direction of the sample liquid (suspension) in the sipper nozzle 30 or in a cross section perpendicular to the axis of the sipper nozzle 30 is denser on an outer side of the cross section 60 (see FIG. 2) than on an inner side of the cross section 60 (denser on a downstream side than on an upstream side with respect to the flow direction 61 of the sample liquid). After the sample liquid (suspension) moves from the sipper nozzle 30 to the flow channel 10, the distribution of the magnetic particles 13 in the proximal region in the cross section perpendicular to the flow direction 61 of the sample liquid (suspension) is denser than that in the distal region. In this way, a distribution of the magnetic particles 13 as illustrated in A of FIG. 8 can be formed in the cross section 60 of the sipper nozzle 30, and a magnetic particle distribution in the flow channel 10 can be formed as illustrated in B of FIG. 8. Therefore, a capture rate of the magnetic particles in the capture region (reaction field electrode 14) can be increased.

The sipper nozzle 30 in Example 1 has a shape like an injection needle. That is, a distal end portion of the sipper nozzle 30 extending from the flow channel 10 in the vertical direction is inclined to form a predetermined angle with a horizontal direction, and an opening of the distal end portion has an elliptical shape. In this case, the predetermined angle is 20 degrees or more.

(iii) The sipper nozzle 30 according to Example 2 includes a plurality of bent portions between a distal end portion thereof and a junction portion (bent portion 80) with the flow channel 10 (see FIG. 14). By adopting the configuration illustrated in Example 2, a distribution of the magnetic particles 13 as illustrated in A of FIG. 8 can be formed in the cross section 60 of the sipper nozzle 30, and a magnetic particle distribution in the flow channel 10 can be formed as illustrated in B of FIG. 8. Therefore, a capture rate of the magnetic particles in the capture region (reaction field electrode 14) can be increased.
(iv) The sipper nozzle 30 according to Example 3 includes the flow contraction portion 82 having a narrow tube diameter between a distal end portion of the sipper nozzle 30 and a junction portion (bent portion 80) with the flow channel 10 (see FIG. 16). Further, a hole may be provided on a tube side surface between a distal end portion of the sipper nozzle 30 and a junction portion (bent portion 80) (see FIG. 17). Further, the sipper nozzle 30 may be configured to include a branch tube that introduces the sample liquid (suspension) from a direction different from a direction of a distal end portion thereof on a tube side surface between the distal end portion and the junction portion (bent portion 80) (see FIG. 18). Furthermore, the sipper nozzle 30 may be configured such that a tube diameter gradually changes from the distal end portion thereof to the junction portion (bent portion 80) (see FIG. 19). By adopting the configuration illustrated in Example 3, a distribution of the magnetic particles 13 as illustrated in A of FIG. 8 can be formed in the cross section 60 of the sipper nozzle 30, and a magnetic particle distribution in the flow channel 10 can be formed as illustrated in B of FIG. 8. Therefore, a capture rate of the magnetic particles in the capture region (reaction field electrode 14) can be increased.
(v) According to Example 4, the flow channel 10 including the capture region (reaction field electrode 14) is placed in a horizontal direction. At this time, the magnetic particle distribution adjustment unit is configured to insert the sipper nozzle 30 extending from the flow channel 10 in the vertical direction into a sample container (suspension container 40) containing the sample liquid (suspension) at a predetermined amount shifted from the center axis of the sample container, and to aspirate the sample liquid (see FIG. 20). Further, according to another mode of Example 4 (FIG. 22), the magnetic particle distribution adjustment unit is configured to insert the sipper nozzle 30 extending from the flow channel 10 in a vertical direction into a sample container (suspension container) having a center axis inclined with respect to the horizontal direction, and to aspirate the sample liquid (suspension) from the sample container. An inclination of the sample container may be achieved by controlling a stage on which the sample container is placed to incline the sample container, or the sample container itself may be configured to have an inclination with respect to the horizontal direction. With such a configuration, a flow (during aspiration) of the sample liquid (suspension) in the sample container can be made asymmetric around the sipper nozzle 30. Therefore, a distribution of the magnetic particles 13 as illustrated in A of FIG. 8 can be formed in the cross section 60 of the sipper nozzle 30, and a magnetic particle distribution in the flow channel 10 can be formed as illustrated in B of FIG. 8. Therefore, a capture rate of the magnetic particles in the capture region (reaction field electrode 14) can be increased.
(vi) According to Example 5, in the sample analyzer (immunoanalyzer 100), the magnetic particle distribution adjustment unit is configured to insert the sipper nozzle 30 extending from the flow channel 10 in a vertical direction into a sample container having an asymmetric shape in a predetermined plane including a center axis of the sample container (suspension container 40) (having an elliptical cross section of the sample container in a horizontal direction or having a protrusion on an inner side surface of the sample container), and to aspirate the sample liquid (suspension) from the sample container. With such a configuration, a flow (during aspiration) of the sample liquid (suspension) in the sample container can be made asymmetric around the sipper nozzle 30. Therefore, a distribution of the magnetic particles 13 as illustrated in A of FIG. 8 can be formed in the cross section 60 of the sipper nozzle 30, and a magnetic particle distribution in the flow channel 10 can be formed as illustrated in B of FIG. 8. Therefore, a capture rate of the magnetic particles in the capture region (reaction field electrode 14) can be increased.
(vii) According to Example 6, in the sample analyzer (immunoanalyzer 100), the flow channel 10 including the capture region (reaction field electrode 14) is placed in a horizontal direction, and the magnetic particle distribution adjustment unit is configured to aspirate the sample liquid (suspension) from a sample container (suspension container 40) using the sipper nozzle 30 extending from the flow channel 10 in a vertical direction, to aspirate a solvent via a solvent supply flow channel (flow channel 90) extending in the horizontal direction (direction opposite to the flow channel 10) from a connection portion (joining portion 85) between the flow channel 10 and the sipper nozzle 30, and to cause the sample liquid and the solvent to join at a junction portion (joining portion 85). In this way, a magnetic particle distribution in the flow channel 10 can be formed as illustrated in B of FIG. 26. Therefore, a capture rate of the magnetic particles in the capture region (reaction field electrode 14) can be increased.
(viii) According to Example 7, in the sample analyzer (immunoanalyzer 100), the magnetic particle distribution adjustment unit includes a sample liquid injection port (suspension injection port 87) provided on an upstream side of the flow channel 10 and for injecting the sample liquid (suspension), and is configured to aspirate the sample liquid injected from the sample injection port of a sample liquid injection flow channel (flow channel 90) to introduce the sample liquid to the capture region (reaction field electrode 14) of the flow channel 10, the sample liquid injection flow channel including, on a top surface of the flow channel, a flow contraction plate 86 that regulates a flow channel width. Accordingly, a magnetic particle distribution in the flow channel 10 can be formed as illustrated in B of FIG. 28. Therefore, a capture rate of the magnetic particles in the capture region (reaction field electrode 14) can be increased.
(ix) According to Example 8, in the sample analyzer (immunoanalyzer 100), the magnetic particle distribution adjustment unit includes a sample liquid injection port (suspension injection port 87) for injecting the sample liquid (suspension) and the solvent injection port 88 for injecting a solvent, which are provided on an upstream side of the flow channel 10, and is configured to aspirate the sample liquid injected from the sample injection port and the solvent injected into the solvent injection port to introduce the sample liquid and the solvent to the capture region (reaction field electrode 14) of the flow channel 10. Accordingly, the magnetic particle distribution in the flow channel 10 can be formed as illustrated in B of FIG. 8. Therefore, a capture rate of the magnetic particles in the capture region (reaction field electrode 14) can be increased.

### Reference Signs List

10 flow channel
11 flow channel top surface wall
12 flow channel bottom surface wall
13 magnetic particle
14 reaction field electrode
15 counter electrode
16, 17 voltage application unit
20 magnet
23 photodetector
30 sipper nozzle
31 arm
32, 33, 34 tube (flow channel)
35 pump
36, 37 valve
38 sipper nozzle distal end portion
40 suspension container
41 reaction unit
42 probe cleaner container
43 buffer solution container
44 cleaning mechanism
45 waste liquid container
50 controller
51, 52, 53 signal line
55a, 55b, 55c, 56a, b, 57 signal line
60 cross section of sipper nozzle 30
61 flow direction in flow channel 10
63 cross section on downstream side
64 cross section on upstream side of flow contraction plate 86
65 cross section on downstream side of flow contraction plate 86
71 protrusion
80 bent portion
82 flow contraction portion
83 hole
84 branch flow channel
85 joining portion
86 flow contraction plate
87 suspension injection port
88 solvent injection port
90 flow channel
91 flow direction of flow channel 90
100 immunoanalyzer (sample analyzer)

## Claims

1. A sample analyzer comprising:
a flow channel including a capture region defined therein and configured to introduce, into the capture region, a sample liquid containing magnetic particles bound to a specific substance;
a supply unit configured to supply the sample liquid to the flow channel;
a capture unit configured to generate a magnetic field and cause the magnetic particles to be adsorbed to the capture region by the magnetic field;
a magnetic particle distribution adjustment unit configured to make, for a distribution of the magnetic particles in a cross section perpendicular to a flow direction of the sample liquid in the capture region of the flow channel, a distribution in a proximal region closer to the capture region than a center axis of the flow channel denser than a distribution in a distal region farther from the central axis as viewed from the capture region;
a measurement unit configured to measure the specific substance adsorbed to the capture region; and
a discharge unit configured to discharge the magnetic particles from the flow channel after measurement by the measurement unit.

2. The sample analyzer according to claim 1, wherein
the flow channel including the capture region is placed in a horizontal direction,
the magnetic particle distribution adjustment unit includes a sample liquid introduction tube extending from the flow channel in a vertical direction, and
the sample liquid introduction tube is configured to have an asymmetric shape on a predetermined plane including a center axis of the sample liquid introduction tube.

3. The sample analyzer according to claim 2, wherein
when the supply unit introduces the sample liquid into the flow channel via the sample liquid introduction tube, a distribution of the magnetic particles in a cross section perpendicular to a flow direction of the sample liquid in the sample liquid introduction tube or an axis of the sample liquid introduction tube is denser on an outer side of the cross section than on an inner side of the cross section, and
after the sample liquid is transferred from the sample liquid introduction tube to the flow channel, the distribution of the magnetic particles in the cross section perpendicular to the flow direction of the sample liquid is denser in the proximal region than in the distal region.

4. The sample analyzer according to claim 3, wherein
when the supply unit introduces the sample liquid into the flow channel via the sample liquid introduction tube, the distribution of the magnetic particles in the cross section perpendicular to the flow direction of the sample liquid in the sample liquid introduction tube or the axis of the sample liquid introduction tube is denser on a downstream side than on an upstream side with respect to a flow direction of the sample liquid in the flow channel.

5. The sample analyzer according to claim 2, wherein
a distal end portion of the sample liquid introduction tube extending from the flow channel in the vertical direction is inclined at a predetermined angle with respect to the horizontal direction, and an opening of the distal end portion has an elliptical shape.

6. The sample analyzer according to claim 5, wherein
the predetermined angle is 20 degrees or more.

7. The sample analyzer according to claim 6, wherein
the predetermined angle is 60 degrees or more.

8. The sample analyzer according to claim 2, wherein
the sample liquid introduction tube includes a plurality of bent portions between a distal end portion and a junction portion with the flow channel.

9. The sample analyzer according to claim 2, wherein
the sample liquid introduction tube includes a flow contraction portion having a narrow tube diameter between a distal end portion and a junction portion with the flow channel.

10. The sample analyzer according to claim 2, wherein
the sample liquid introduction tube has a hole provided on a tube side surface between a distal end portion and a junction portion with the flow channel.

11. The sample analyzer according to claim 2, wherein
the sample liquid introduction tube includes a branch tube that introduces the sample liquid from a direction different from a direction of a distal end portion on a tube side surface between the distal end portion and a junction portion with the flow channel.

12. The sample analyzer according to claim 2, wherein
the sample liquid introduction tube is configured such that a tube diameter gradually changes from a distal end portion to a junction portion with the flow channel.

13. The sample analyzer according to claim 1, wherein
the flow channel including the capture region is placed in a horizontal direction, and
the magnetic particle distribution adjustment unit inserts a sample liquid introduction tube extending from the flow channel in a vertical direction into a sample container containing the sample liquid at a predetermined amount shifted from a center axis of the sample container, and aspirates the sample liquid.

14. The sample analyzer according to claim 1, wherein
the flow channel including the capture region is placed in a horizontal direction, and
the magnetic particle distribution adjustment unit inserts a sample liquid introduction tube extending from the flow channel in a vertical direction into a sample container having a center axis inclined with respect to the horizontal direction, and aspirates the sample liquid from the sample container.

15. The sample analyzer according to claim 14, wherein
the magnetic particle distribution adjustment unit controls a stage on which the sample container containing the sample liquid is placed to incline the sample container.

16. The sample analyzer according to claim 14, wherein
the sample container itself is configured to have an inclination with respect to the horizontal direction.

17. The sample analyzer according to claim 1, wherein
the flow channel including the capture region is placed in a horizontal direction, and
the magnetic particle distribution adjustment unit inserts a sample liquid introduction tube extending from the flow channel in the vertical direction into a sample container having an asymmetric shape on a predetermined plane including a center axis, and aspirates the sample liquid from the sample container.

18. The sample analyzer according to claim 17, wherein
a cross section of the sample container in the horizontal direction has an elliptical shape.

19. The sample analyzer according to claim 17, wherein
the sample container has a protrusion on an inner side surface of the sample container.

20. The sample analyzer according to claim 1, wherein
the flow channel including the capture region is placed in a horizontal direction, and
the magnetic particle distribution adjustment unit aspirates the sample liquid from a sample container containing the sample liquid using a sample liquid introduction tube extending from the flow channel in a vertical direction, aspirates a solvent via a solvent supply flow channel extending in the horizontal direction from a connection portion between the flow channel and the sample liquid introduction tube, and causes the sample liquid and the solvent to join at the connection portion.

21. The sample analyzer according to claim 1, wherein
the magnetic particle distribution adjustment unit includes a sample liquid injection port provided on an upstream side of the flow channel and for injecting the sample liquid, and is configured to aspirate the sample liquid injected from the sample liquid injection port of a sample liquid injection flow channel to introduce the sample liquid to the capture region of the flow channel, the sample liquid injection flow channel including, on a top surface of the flow channel, a flow contraction portion that regulates a flow channel width.

22. The sample analyzer according to claim 1, wherein
the magnetic particle distribution adjustment unit includes a sample liquid injection port for injecting the sample liquid and a solvent injection port for injecting a solvent, which are provided on an upstream side of the flow channel, and is configured to aspirate the sample liquid injected from the sample liquid injection port and the solvent injected into the solvent injection port to introduce the sample liquid and the solvent to the capture region of the flow channel.
